# FASCICULE DE BREVET EUROPEEN

(11) **EP 2 185 525 B1**
(45) Date de publication et mention de la délivrance du brevet: **14.12.2016**
(21) Numéro de dépôt: 08828371.8
(22) Date de dépôt: 15.07.2008
(51) Int. Cl.: A61K 31/415, A61K 31/4439, A61P 25/00

(54) **DÉRIVÉS DE PYRAZOLE 3,5-CARBOXYLATES, LEUR PRÉPARATION ET LEUR APPLICATION EN THÉRAPEUTIQUE**
HERSTELLUNG VON PYRAZOL-3,5-CARBOXYLAT-DERIVATEN UND IHRE THERAPEUTISCHE ANWENDUNG
PYRAZOLE 3,5 CARBOXYLATE DERIVATIVES PREPARATION AND THERAPEUTIC APPLICATION THEREOF

(30) Priorité: 16.07.2007 FR 0705123; 16.07.2007 US 949950 P
(43) Date de publication de la demande: 19.05.2010
(73) Titulaire: SANOFI, 75008 Paris (FR)
(72) Inventeur: FORICHER, Yann, F-75013 Paris (FR); SMRCINA, Martin, F-75013 Paris (FR); VAN DORSSELAER, Viviane, F-75013 Paris (FR); WEBER, Fabienne, F-75013 Paris (FR)
(74) Mandataire: Lavoix
(86) Numéro de dépôt international: PCT/FR2008/001028
(87) Numéro de publication internationale: WO 2009/027601

(56) Documents cités:
- WO-A-03/040096
- HAQUE T S ET AL: "Parallel synthesis of potent, pyrazole-based inhibitors of Helicobacter pylori dihydroorotate dehydrogenase" JOURNAL OF MEDICINAL CHEMISTRY 10 OCT 2002 UNITED STATES, vol. 45, no. 21, 10 octobre 2002 (2002-10-10), pages 4669-4678, XP002470864 ISSN: 0022-2623

## Description

La présente invention se rapporte à des dérivés de pyrazole 3,5-carboxylates, à leur préparation et à leurs applications en thérapeutique.

Il a été trouvé que ces composés, dérivés de pyrazole 3,5-carboxylates, sont des inhibiteurs de la β-secrétase BACE1 (β-site Amyloid precursor protein Cleavage Enzyme subtype 1).

La demande WO 03/040096 décrit des composés de formule suivante : 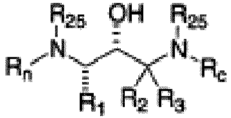 utiles comme inhibiteur de la formation de peptide β-amyloïde β-A4.

La présente demande décrit des composés répondant à la formule (I) : 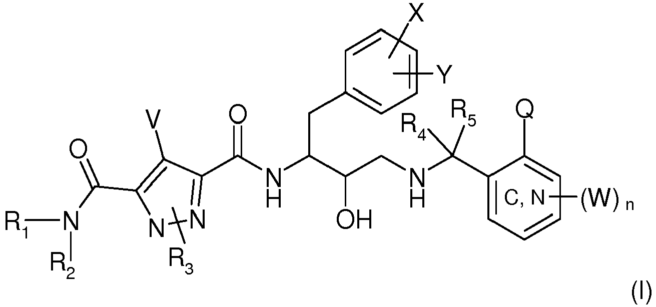 dans laquelle
- R₁ représente un atome d'hydrogène, un groupe C₃₋₆-cycloalkyle, C₁₋₆-alkyle, ledit C₁₋₆-alkyle étant éventuellement substitué par un ou plusieurs groupes choisis parmi un hydroxy, C₁₋₆-alcoxy ;
- R₂ représente un atome d'hydrogène, un groupe C₁₋₁₀-alkyle, un aryle plus particulièrement un phényle, lesdits groupe C₁₋₁₀-alkyle et phényle étant éventuellement substitués par un ou plusieurs groupes choisis parmi hydroxy, C₁₋₆-alkyle, C₁₋₆-alcoxy ;
   étant entendu que l'un au moins de R₁ ou de R₂ est différent d'un atome d'hydrogène ;
   . ou R₂ représente un groupe NRₐR_{b} dans lequel :
   Rₐ et R_{b} représentent, indépendamment l'un de l'autre, un atome d'hydrogène, un groupe C₁₋₆-alkyle, un groupe aryle, C₁₋₆-alkylène-aryle ou C₁₋₆-alkylène-hétéroaryle, lesdits groupes C₁₋₆-alkyle, aryle, C₁₋₆-alkylène-aryle ou C₁₋₆-alkylène-hétéroaryle étant éventuellement substitués par un ou plusieurs groupes choisis parmi hydroxy, C₁₋₆-alkyle, C₁₋₆-alcoxy, halogène, CF₃ ou OCF₃,
      - ou bien Rₐ et R_{b} forment ensemble avec l'atome d'azote auquel ils sont rattachés un hétérocycle, ledit hétérocyle étant éventuellement substitué par un ou plusieurs groupes choisis parmi C₁₋₆-alkyle, C₁₋₆-alkylène-O-C₁₋₆-alkyle ou C₁₋₆-alcoxy, halogène, CF₃ ou OCF₃;
      - ou bien R₁ et R₂ forment ensemble avec l'atome d'azote auquel ils sont rattachés un groupe hétérocycle en particulier un groupe pipéridinyle ou morpholinyle, ledit groupe hétérocycle étant éventuellement substitué par un ou plusieurs groupes choisis parmi C₁₋₆-alkyle, C₁₋₆-alcoxy, C₁₋₆-alkylène-O-C₁₋₆-alkyle ;
- R₃ représente un groupe C₁₋₆-alkyle en particulier un groupe éthyle ou butyle, ou un benzyle ;
- R₄ et R₅ représentent indépendamment l'un de l'autre un atome d'hydrogène, un groupe C₁₋₆-alkyle,
   - ou bien R₄ et R₅ forment ensemble, avec l'atome de carbone auquel ils sont reliés un cycle spiro contenant de 3 à 5 atomes de carbone ; en particulier trois atomes de carbone ;
- Q représente un atome d'hydrogène, un groupe C₁₋₃-alkyle ;
   - ou bien Q et R₅ forment ensemble et avec la chaîne carbonée à laquelle ils sont rattachés, un cycle carboné saturé comportant 5 ou 6 atomes de carbone, ledit cycle étant éventuellement substitué par un ou deux groupes R, le ou les groupes R représentant indépendamment l'un de l'autre choisis parmi un atome d'halogène, un groupe C₁₋₃-alkyle, OCHF₂, OCF₃, CF₃ ou C₁₋₃-alcoxy ;
   - ou bien Q et R₅ forment ensemble et avec la chaîne carbonée à laquelle ils sont rattachés, un cycle saturé comportant 4 ou 5 atomes de carbone et un atome d'oxygène, ledit cycle étant éventuellement substitué par un ou deux groupes R, le ou les groupes R représentant indépendamment l'un de l'autre choisis parmi un atome d'halogène, un groupe C₁₋₃-alkyle, OCHF₂, OCF₃, CF₃ ou C₁₋₃-alcoxy ;
- X et Y représentent indépendamment l'un de l'autre un atome d'hydrogène, d'halogène, un groupe-O-C₂₋₆-alkényle, ou -O-benzyle ;
- n représente un nombre entier choisi parmi 1 ou 2 ;
- V représente un atome d'hydrogène ; et
- W représente un atome d'halogène, un groupe CF₃, C₁₋₆-alkyle, -COOH, C₁₋₆-alcoxy ;
   étant entendu que lorsque n est 2 ou 3, les deux ou trois groupes W représentent, indépendamment les uns des autres, les définitions mentionnées ci-dessus.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates et/ou de solvats, à savoir sous forme d'associations ou de combinaisons avec une ou plusieurs molécules d'eau et/ou de solvant. De tels hydrates et solvats font également partie de l'invention.

Dans le cadre de la présente invention, on entend par :
- un atome d'halogène : un fluor, un chlore, un brome ou un iode ;
- un groupe C₁₋₆-alkyle : un groupe aliphatique monovalent saturé, linéaire ou ramifié pouvant comprendre 1 à 6 atomes de carbone. A titre d'exemples, on peut citer les groupes méthyle, éthyle, propyle, isopropyle, butyle, isobutyle, *tert*-butyle, pentyle ; cette définition étant également valable pour les groupes C₁₋₃-alkyle, C₁₋₄-alkyle, C₁₋₁₀-alkyle par exemple pour lesquels seul change le nombre de carbone possible ;
- un groupe C₁₋₆-alkylène : un groupe aliphatique divalent saturé, linéaire ou ramifié pouvant comporter de 1 à 6 atomes de carbone. A titre d'exemple, on peut citer les groupes méthylènyle (-CH₂-), un éthylènyle (-CH₂CH₂-), un 1-méthyléthylènyle (-CH(CH₃)CH₂-), un propylènyle (-CH₂CH₂CH₂-) ;
- un groupe C₁₋₆-alcoxy : un radical alkyle-O- où le groupe alkyle est tel que précédemment défini ; de même pour le groupe C₁₋₃-alcoxy pour lequel seul le nombre de carbone possible change ;
- un groupe C₂₋₆-alkényle : un groupe aliphatique mono- ou poly-insaturé, linéaire ou ramifié, pouvant comprendre de 2 à 6 atomes de carbone et par exemple une ou deux insaturations éthyléniques. Par exemple, on peut citer les groupes éthényle, propényle ;
- un groupe hétérocycle : un groupe cyclique saturé de 4 à 7 chaînons comportant un à plusieurs hétéroatomes tels qu'un atome d'azote, d'oxygène ou de soufre. A titre d'exemples, on peut citer les groupes pyrrolidinyle, pipéridinyle, tétrahydropyranyle, pipéridonyle, morpholinyle, pipérazinyle, *N-*méthyl-pipérazinyle, homomorpholinyle, oxétanyle ;
- un groupe aryle est un système aromatique monocyclique ou polycyclique comprenant de 6 à 14 atomes de carbone, de préférence de 6 à 10 atomes de carbone. Lorsque le système est polycyclique, au moins un des cycles est aromatique. A titre d'exemples, on peut citer les groupes phényle, naphtyle, tétrahydronaphtyle, indanyle ;
- un groupe hétéroaryle, un groupe aromatique cyclique comprenant entre 1 et 6 atomes de carbone et entre 1 et 4 hétéroatomes, tels que l'azote, l'oxygène ou le soufre. A titre d'exemples de groupes hétéroaromatiques, on peut citer les groupes oxazolyle, oxadiazolyle, tétrazolyle, thiazolyle, thiadiazolyle, thiényle, imidazolyle, triazolyle, pyridyle, furanyle, isoxazolyle, pyrrolyle, pyrazolyle, pyrimidinyle, pyridazinyle, les atomes d'azote pouvant être, selon les cas, sous forme de *N-*oxydes ;
- « deux groupes R portés par le même atome de carbone, forment ensemble un groupe oxo », le groupe tel que : 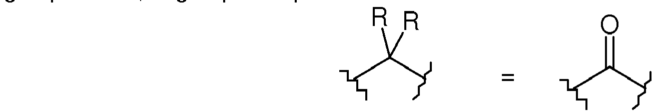
- le groupe aromatique : 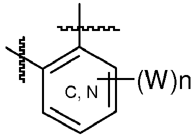 est tel que un des atomes de carbone du cycle aromatique peut être remplacé par un atome d'azote (dans la position où il n'y a pas de substituant W) ; dans la présente description, on utilise indifféremment ce groupe aromatique ou celui-ci-dessous, 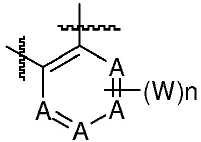 dans lesquels tous les atomes A sont des atomes de carbone, l'un des atomes A pouvant néanmoins être un atome d'azote si cet atome ne porte pas le substituant W.
- « Q et R₅ forment ensemble et avec la chaîne carbonée à laquelle ils sont rattachés, un cycle carboné saturé comportant 5 ou 6 atomes de carbone», représente un groupe du type : 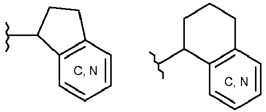 par exemple.
- « Q et R₅ forment ensemble et avec la chaîne carbonée à laquelle ils sont rattachés, un cycle saturé comportant 4 ou 5 atomes de carbone et un hétéroatome », représente un groupe du type : 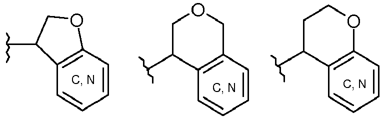 par exemple.
- « R₄ et R₅ forment ensemble, avec l'atome de carbone auquel ils sont reliés un cycle spiro contenant de 3 à 6 atomes de carbone», représente un groupe du type : 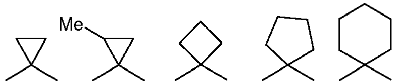 par exemple.
- « R₄ et R₅ forment ensemble et avec la chaîne carbonée à laquelle ils sont rattachés, un cycle saturé comportant 3 ou 5 atomes de carbone et un atome d'oxygène, ledit cycle étant éventuellement substitué par un ou plusieurs groupe R, représentant indépendamment l'un de l'autre un atome d'halogène, un groupe C₁₋₃-alkyle, C₁₋₃-alcoxy », représente un groupe du type : 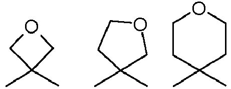 par exemple

Dans ce qui suit, les nomenclatures des composés suivent les règles IUPAC.

Parmi les composés de formule (I), un groupe de composés est constitué par les composés de formule (Iter) objets de l'invention : 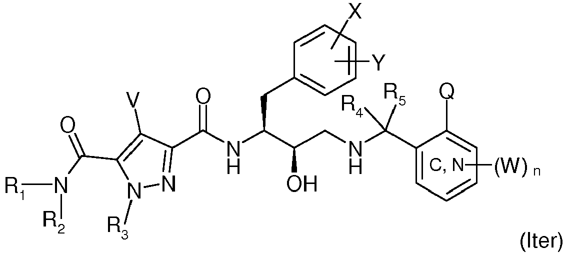 dans laquelle R₁, R₂, R₃, R₄, R₅ X, Y, n, Q, V et W ont les mêmes définitions que les composés de formule (I).

Parmi les composés de formule (I) objets de l'invention, un autre groupe de composés est constitué par les composés de formule (Iquater) : 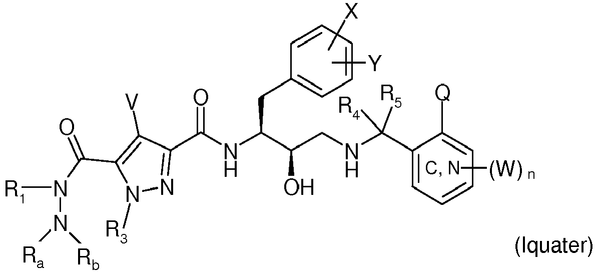 dans laquelle Rₐ, R_{b}, R₁, R₂, R₃, R₄, R₅ X, Y, n, Q, V et W ont les mêmes définitions que les composés de formule (I).

Parmi les composés objets de l'invention, d'autres groupes de composés sont constitués par les composés de formules (I), (I ter) ou (I quater) pour lesquels le carbone portant R₄ est asymmétrique et a la configuration suivante quand Q et R₅ forment ensemble et avec la chaîne carbonée à laquelle ils sont rattachés, un cycle : 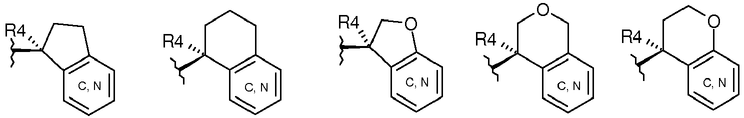 par exemple. Un groupe de l'invention est également constitué par les composés de formule (I) dans lesquels les groupes X et Y sont de préférence en position méta ou para sur le phényle auquel ils sont rattachés.

Parmi les composés de formule (I) objets de l'invention, on peut notamment citer les composés choisis parmi :
- *N*³-[(1*S*,2*R*)-1-benzyl-2-hydroxy-3-{[3-(trifluorométhyl)benzyl]amino}propyl]-1-éthyl-*N*⁵,*N*⁵-dipropyl-1*H-*pyrazole-3,5-dicarboxamide ;
- *N*³-[(1*S*,2*R*)-1-benzyl-2-hydroxy-3-{[3-(méthoxy)benzyl]amino}propyl]-1-éthyl-*N*⁵,*N*⁵-dipropyl-1*H-*pyrazole-3,5-dicarboxamide ;
- 1-éthyl-*N*³-[(1*S*,2*R*)-2-hydroxy-1-[3-(prop-2-én-1-yloxy)benzyl]-3-({1-[3-(trifluorométhyl)phényl]cyclopropyl}amino)propyl]-*N*⁵,N⁵-dipropyl-1*H*-pyrazole-3,5-dicarboxamide
- *N*³-[(1*S*,2*R*)-1-[4-(benzyloxy)benzyl]-2-hydroxy-3-({1-[3-(trifluorométhyl)phényl]cyclopropyl}amino)propyl]-1-éthyl-*N*⁵,*N*⁵-dipropyl-1*H*-pyrazole-3,5-dicarboxamide ;
- *N³-*[(1*S,*2*R*)*-*1*-*(3,5-difluorobenzyl)-2-hydroxy-3-{[3-(trifluorométhyl)benzyl]amino}propyl]-1-éthyl-*N*⁵,*N*⁵-dipropyl-1*H*-pyrazole-3,5-dicarboxamide ;
- 1-benzyl-*N*³-[(1*S*,2*R*)-1-benzyl-2-hydroxy-3-{[3-(trifluorométhyl)benzyl]amino}propyl]-*N*⁵,*N*⁵-dipropyl-1*H*-pyrazole-3,5-dicarboxamide ;
- *N*³-[(1*S*,2*R*)-1-benzyl-2-hydroxy-3-{[3-(trifluorométhyl)benzyl]amino}propyl]-*N*⁵,*N*⁵-dibutyl-1-éthyl-1*H*-pyrazole-3,5-dicarboxamide ;
- *N*³-[(1*S*,2*R*)-1-benzyl-2-hydroxy-3-{[3-(trifluorométhyl)benzyl]amino}propyl]-1-éthyl-*N*⁵-(1-propylbutyl)-1*H*-pyrazole-3,5-dicarboxamide ;
- *N*³-[(1*S*,2*R*)-1-benzyl-2-hydroxy-3-{[3-(trifluorométhyl)benzyl]amino}propyl]-*N*⁵-cyclopropyl-1-éthyl-1*H*-pyrazole-3,5-dicarboxamide ;
- *N*³-[(1*S*,2*R*)-1-benzyl-2-hydroxy-3-{[3-(trifluorométhyl)benzyl]amino}propyl]-*N*⁵-butyl-*N*⁵-cyclopropyl-1-éthyl-1*H*-pyrazole-3,5-dicarboxamide ;
- *N*³-[(1*S*,2*R*)-2-benzyl-2-hydroxy-3-{[3-(trifluorométhyl)benzyl]amino}propyl]-*N*⁵-butyl-1-éthyl-1*H*-pyrazole-3,5-dicarboxamide ;
- *N*³-[(1*S*,2*R*)-1-benzyl-2-hydroxy-3-{[3-(trifluorométhyl)benzyl]amino}propyl]-1-éthyl-*N*⁵-hexyl-1*H*-pyrazole-3,5-dicarboxamide ;
- *N*³-[(1*S*,2*R*)-1-benzyl-2-hydroxy-3-{[3-(trifluorométhyl)benzyl]amino}propyl]-1-éthyl-*N*⁵-méthyl-*N*⁵-pentyl-1*H*-pyrazole-3,5-dicarboxamide ;
- *N*³-[(1*S*,2*R*)-1-benzyl-2-hydroxy-3-{[3-(trifluorométhyl)benzyl]amino}propyl]-*N*⁵-cyclopropyl-1-éthyl-*N*⁵-hexyl-1*H*-pyrazole-3,5-dicarboxamide ;
- *N*³-[(1*S*,2*R*)-1-benzyl-2-hydroxy-3-{[3-(trifluorométhyl)benzyl]amino}propyl]-1-éthyl-*N*⁵-heptyl-*N*⁵-méthyl-1*H*-pyrazole-3,5-dicarboxamide ;
- *N*³-[(1*S*,2*R*)-1-benzyl-2-hydroxy-3-{[3-(trifluorométhyl)benzyl]amino}propyl]-1-éthyl-*N*⁵-hexyl-*N*⁵-méthyl-1*H*-pyrazole-3,5-dicarboxamide ;
- *N³-*[(1*S*,2*R*)-1-(3,5-difluorobenzyl)-2-hydroxy-3-{[(1*S*)-7-méthoxy-1,2,3,4-tétrahydronaphtalén-1-yl]amino}propyl]-1-éthyl-*N*⁵-hexyl-*N*⁵-méthyl-1*H*-pyrazole-3,5-dicarboxamide ;
- *N*³-[(1*S*,2*R*)-1-(3,5-difluorobenzyl)-2-hydroxy-3-{[(1*S*)-7-méthoxy-1,2,3,4-tétrahydronaphtalén-1-yl]amino}propyl]-*N*⁵-(3-éthoxypropyl)-1-éthyl-*N*⁵-méthyl-1*H-*pyrazole-3,5-dicarboxamide
- *N*⁵-cyclopropyl-*N*³-[(1*S*,2*R*)-1-(3,5-difluorobenzyl)-2-hydroxy-3-({1-[3-(trifluorométhyl)phényl]cyclopropyl}amino)propyl]-1-éthyl-*N*⁵-hexyl-1*H*-pyrazole-3,5-dicarboxamide ;
- *N*³-[(1*S*,2*R*)-1-(3,5-difluorobenzyl)-2-hydroxy-3-({1-[3-(trifluorométhyl)phényl]cyclopropyl}amino)propyl]-1-éthyl-*N*⁵-heptyl-*N⁵*-méthyl-1*H-*pyrazole-3,5-dicarboxamide ;
- *N*³-[(1*S*,2*R*)-1-(3,5-difluorobenzyl)-2-hydroxy-3-{[(1*S*)-7-méthoxy-1,2,3,4-tétrahydronaphtalén-1-yl]amino}propyl]-1-éthyl-*N*⁵-heptyl-*N*⁵-méthyl-1*H*-pyrazole-3,5-dicarboxamide ;
- *N*³-[(1*S*,2*R*)-1-benzyl-2-hydroxy-3-{[3-(trifluorométhyl)benzyl]amino}propyl]-*N*⁵-cyclopropyl-1-éthyl-*N*⁵-(3-hydroxypropyl)-1*H-*pyrazole-3,5-dicarboxamide ;
- *N*³-[(1*S*,2*R*)-1-benzyl-2-hydroxy-3-{[3-(trifluorométhyl)benzyl]amino}propyl]-*N*⁵-cyclopropyl-1-éthyl-*N*⁵-(4-hydroxybutyl)-1*H*-pyrazole-3,5-dicarboxamide ;
- *N*³-[(1*S*,2*R*)-1-benzyl-2-hydroxy-3-{[3-(trifluorométhyl)benzyl]amino}propyl]-*N*⁵-cyclopropyl-1-éthyl-*N*⁵-(5-hydroxypentyl)-1*H*-pyrazole-3,5-dicarboxamide ;
- *N*³-[(1*S*,2*R*)-1-benzyl-2-hydroxy-3-{[3-(trifluorométhyl)benzyl]amino}propyl]-*N*⁵-cyclopropyl-*N*⁵-(3-éthoxypropyl)-1-éthyl-1*H*-pyrazole-3,5-dicarboxamide ;
- *N*⁵-cyclopropyl-*N*³-[(1*S*,2*R*)-1-(3,5-difluorobenzyl)-2-hydroxy-3-{[(1*S*)-7-méthoxy-1,2,3,4-tétrahydronaphtalén-1-yl]amino}propyl]-*N*⁵-(3-éthoxypropyl)-1-éthyl-1*H-*pyrazole-3,5-dicarboxamide
- *N*³-[(1*S*,2*R*)-1-benzyl-2-hydroxy-3-{[3-(trifluorométhyl)benzyl]amino}propyl]-1-éthyl-*N*⁵-(2-éthylphényl)-1*H*-pyrazole-3,5-dicarboxamide ;
- *N*³-[(1*S*,2*R*)-1-benzyl-2-hydroxy-3-{[3-(trifluorométhyl)benzyl]amino}propyl]-*N*⁵-butyl-1-éthyl-*N*⁵-(2-éthylphényl)-1*H*-pyrazole-3,5-dicarboxamide ;
- *N*-[(1*S*,2*R*)-1-benzyl-2-hydroxy-3-{[3-(trifluorométhyl)benzyl]amino}propyl]-1-éthyl-5-(morpholin-4-ylcarbonyl)-1*H*-pyrazole-3-carboxamide ;
- *N*-[(1*S*,2*R*)-1-benzyl-2-hydroxy-3-{[3-(trifluorométhyl)benzyl]amino}propyl]-1-éthyl-5-[(5-éthyl-2-méthylpipéridin-1-yl)carbonyl]-1*H*-pyrazole-3-carboxamide ;
- *N*-[(1*S*,2*R*)-1-benzyl-2-hydroxy-3-{[3-(trifluorométhyl)benzyl]amino}propyl]-1-butyl-5-[(2-propylpipéridin-1-yl)carbonyl]-1*H*-pyrazole-3-carboxamide ;
- 1-benzyl-*N*-[(1*S*,2*R*)-1-benzyl-2-hydroxy-3-{[3-(trifluorométhyl)benzyl]amino}propyl]-5-[(2-propylpipéridin-1-yl)carbonyl]-1*H*-pyrazole-3-carboxamide ;
- *N*-[(1*S*,2*R*)-1-benzyl-2-hydroxy-3-{[3-(trifluorométhyl)benzyl]amino}propyl]-1-éthyl-5-[(2-propylpipéridin-1-yl)carbonyl]-1*H*-pyrazole-3-carboxamide ;
- *N*-[(1*S*,2*R*)-1-benzyl-2-hydroxy-3-{[3-(trifluorométhyl)benzyl]amino}propyl]-1-éthyl-5-{[(2*S*)-2-propylpipéridin-1-yl]carbonyl}-1*H*-pyrazole-3-carboxamide ;
- *N*-[(1*S*,2*R*)-1-benzyl-2-hydroxy-3-{[3-(trifluorométhyl)benzyl]amino}propyl]-1-éthyl-5-{[(2*R*)-2-propylpipéridin-1-yl]carbonyl}-1*H*-pyrazole-3-carboxamide ;
- *N*-[(1*S*,2*R*)-1-benzyl-3-(benzylamino)-2-hydroxypropyl]-1-éthyl-5-[(2-propylpipéridin-1-yl)carbonyl]-1*H*-pyrazole-3-carboxamide ;
- *N*-[(1*S*,2*R*)-1-benzyl-3-{[4-fluoro-3-(trifluorométhyl)benzyl]amino}-2-hydroxypropyl]-1-éthyl-5-[(2-propylpipéridin-1-yl)carbonyl]-1*H*-pyrazole-3-carboxamide ;
- *N*-{(1*S*,2*R*)-1-benzyl-3-[(4-fluorobenzyl)amino]-2-hydroxypropyl}-1-éthyl-5-[(2-propylpipéridin-1-yl)carbonyl]-1*H*-pyrazole-3-carboxamide ;
- *N*-{(1*S*,2*R*)-1-benzyl-3-[(3,5-difluorobenzyl)amino]-2-hydroxypropyl}-1-éthyl-5-[(2-propylpipéridin-1-yl)carbonyl]-1*H*-pyrazole-3-carboxamide ;
- *N*-{(1*S*,2*R*)-1-benzyl-3-[(3-bromobenzyl)amino]-2-hydroxypropyl}-1-éthyl-5-[(2-propylpipéridin-1-yl)carbonyl]-1*H*-pyrazole-3-carboxamide ;
- *N*-{(1*S*,2*R*)-1-benzyl-2-hydroxy-3-[(3-méthylbenzyl)amino]propyl}-1-éthyl-5-[(2-propylpipéridin-1-yl)carbonyl]-1*H*-pyrazole-3-carboxamide ;
- *N*-{(1*S*,2*R*)-1-benzyl-3-[(4-fluoro-3-méthylbenzyl)amino]-2-hydroxypropyl}-1-éthyl-5-[(2-propylpipéridin-1-yl)carbonyl]-1*H*-pyrazole-3-carboxamide ;
- acide 4-[({(2*R*,3*S*)-3-[({1-éthyl-5-[(2-propylpipéridin-1-yl)carbonyl]-1*H-*pyrazol-3-yl}carbonyl)amino]-2-hydroxy-4-phénylbutyl}amino)méthyl]benzoïque ;
- *N*-[(1*S*,2*R*)-1-benzyl-3-{[3,5-bis(trifluorométhyl)benzyl]amino}-2-hydroxypropyl]-1-éthyl-5-[(2-propylpipéridin-1-yl)carbonyl]-1*H*-pyrazole-3-carboxamide ;
- *N*-{(1*S*,2*R*)-1-benzyl-2-hydroxy-3-[(3-méthoxybenzyl)amino]propyl}-1-éthyl-5-[(2-propylpipéridin-1-yl)carbonyl]-1*H*-pyrazole-3-carboxamide ;
- *N*-{(1*S*,2*R*)-1-benzyl-2-hydroxy-3-[(pyridin-3-ylméthyl)amino]propyl}-1-éthyl-5-[(2-propylpipéridin-1-yl)carbonyl]-1*H*-pyrazole-3-carboxamide ;
- *N*-[(1*S*,2*R*)-1-(3,5-difluorobenzyl)-2-hydroxy-3-{[3-(trifluorométhyl)benzyl]amino}propyl]-1-éthyl-5-[(2-propylpipéridin-1-yl)carbonyl]-1*H-*pyrazole-3-carboxamide ;
- *N*-{(1*S*,2*R*)-1-benzyl-2-hydroxy-3-[(1-méthyl-1-phényléthyl)amino]propyl}-1-éthyl -5-[(2-propylpipéridin-1-yl)carbonyl]-1*H*-pyrazole-3-carboxamide ;
- *N*-{(1*S*,2*R*)-1-benzyl-2-hydroxy-3-({1-méthyl-1-[3-(trifluorométhyl)phényl]éthyl}amino)propyl}-1-éthyl-5-[(2-propylpipéridin-1-yl)carbonyl]-1*H-*pyrazole-3-carboxamide ;
- *N*-{(1*S*,2*R*)-1-(3,5-difluorobenzyl)-2-hydroxy-3-({1-méthyl-1-[3-(trifluorométhyl)phényl]éthyl}amino)propyl}-1-éthyl-5-[(2-propylpipéridin-1-yl)carbonyl]-1*H-*pyrazole-3-carboxamide ;
- *N*-{(1*S*,2*R*)-1-benzyl-2-hydroxy-3-({1-[3-(trifluorométhyl)phényl]cyclopropyl}amino)propyl}-1-éthyl-5-[(2-propylpipéridin-1-yl)carbonyl]-1*H*-pyrazole-3-carboxamide ;
- *N*-{(1*S*,2*R*)-1-[3-(prop-2-én-1-yloxy)benzyl]-2-hydroxy-3-({1-[3-(trifluorométhyl)phényl]cyclopropyl}amino)propyl}-1-éthyl-5-[(2-propylpipéridin-1-yl)carbonyl]-1*H*-pyrazole-3-carboxamide ;
- *N*-{(1*S*,2*R*)-1-(3,5-difluorobenzyl)-2-hydroxy-3-({1-[3-(trifluorométhyl)phényl]cyclopropyl}amino)propyl}-1-éthyl-5-[(2-propylpipéridin-1-yl)carbonyl]-1*H*-pyrazole-3-carboxamide ;
- *N*-{(1*S*,2*R*)-1-benzyl-2-hydroxy-3-({(1*R*)-1-[3-méthoxyphényl]propyl}amino)propyl}-1-éthyl-5-[(2-propylpipéridin-1-yl)carbonyl]-1*H-*pyrazole-3-carboxamide ;
- *N*-{(1*S*,2*R*)-1-benzyl-2-hydroxy-3-({(1*R*)-1-[3,5-bis(trifluorométhyl) phényl]éthyl}amino)propyl}-1-éthyl-5-[(2-propylpipéridin-1-yl)carbonyl]-1*H*-pyrazole-3-carboxamide ;
- *N*-{(1*S*,2*R*)-1-benzyl-2-hydroxy-3-({(1*R*)-1-[3-méthoxyphényl]éthyl}amino)propyl}-1-éthyl-5-[(2-propylpipéridin-1-yl)carbonyl]-1*H-*pyrazole-3-carboxamide ;
- *N*-{(1*S*,2*R*)-1-benzyl-2-hydroxy-3-({(1*R*)-1-[3-(trifluorométhyl) phényl]éthyl}amino)propyl}-1-éthyl-5-[(2-propylpipéridin-1-yl)carbonyl]-1*H*-pyrazole-3-carboxamide ;
- *N*-{(1*S*,2*R*)-1-benzyl-2-hydroxy-3-([(1*R*)-7-méthoxy-1,2,3,4-tétrahydronaphtalén-1-yl]amino)propyl}-1-éthyl-5-[(2-propylpipéridin-1-yl)carbonyl]-1*H-*pyrazole-3-carboxamide ;
- *N*-{(1*S*,2*R*)-1-benzyl-2-hydroxy-3-([(1*R*)-6-méthoxy-2,3-dihydro-1*H*-indén-1-yl]amino)propyl}-1-éthyl-5-[(2-propylpipéridin-1-yl)carbonyl]-1*H*-pyrazole-3-carboxamide ;
- *N*{(1*S*,2*R*)-1-benzyl-2-hydroxy-3-({(1*S*)-1-[3-méthoxyphényl]éthyl}amino)propyl}-1-éthyl-5-[(2-propylpipéridin-1-yl)carbonyl]-1*H-*pyrazole-3-carboxamide ;
- *N*-{(1*S*,2*R*)-1-benzyl-2-hydroxy-3-({(1*S*)-1-[3-méthoxyphényl]propyl}amino)propyl}-1-éthyl-5-[(2-propylpipéridin-1-yl)carbonyl]-1*H-*pyrazole-3-carboxamide ;
- *N*-{(1*S*,2*R*)-1-benzyl-2-hydroxy-3-([(1*S*)-6-méthoxy-2,3-dihydro-1*H*-indén-1-yl]amino )propyl}-1-éthyl-5-[(2-propylpipéridin-1-yl)carbonyl]-1*H*-pyrazole-3-carboxamide ;
- *N*-{(1*S*,2*R*)-1-benzyl-2-hydroxy-3-([(1*S*)-7-méthoxy-1,2,3,4-tétrahydronaphtalén-1-yl]amino)propyl}-1-éthyl-5-[(2-propylpipéridin-1-yl)carbonyl]-1*H-*pyrazole-3-carboxamide ;
- *N*{(1*S*,2*R*)-1-(3,5-difluorobenzyl)-2-hydroxy-3-([(1*S*)-7-méthoxy-1,2,3,4-tétrahydronaphtatén-1-yl]amino)propyl}-1-éthyl-5-[(2-propylpipéridin-1-yl)carbonyl]-1*H-*pyrazole-3-oerboxamide ;
- *N*-[(1*S*,2*R*)-1-(3,5-difluorobenzyl)-2-hydroxy-3-{[(1*S*)-7-(trifluorométhyl)-1,2,3,4-tëtrahydronaphtalén-1-yl]amino}propyl]-1-éthyl-5-[(2-propylpipéridin-1-yl)carbonyl]-1*H*-pyrazole-3-carboxamide
- *N*-[(1*S*,2*R*)-1-benzyl-2-hydroxy-3-{[3-(trifluorométhyl)benzyl]amino}propyl]-1-éthyl-5-{[2-(mëthoxyméthyl)pipéridin-1-yl]carbonyl}-1*H* pyrazole-3-carboxamide ;
- *N-*[(1*S*,2*R*)-1-(3,5-difluorobenzyl-2-hydroxy-3-{[3-(trifluorométhyl)benzyl]amino}propyl]-1-éthyl-5-{[2-(méthoxyméthyl)pipéridin-1-yl]carbonyl}-1*H*-pyrazole-3-carboxamide ;
- *N*³-[(1*S*,2*R*)-1-benzyl-2-hydroxy-3-{[3-(trifluoromëthyl)benzyl]amino}propyl]-*N*⁵-(2,6-diméthylpipéridin-1-yl)-1-éthyl-1*H*-pyrazole-3,5-dicarboxamide ;
- 5-(2-benzyl-2-éthylhydrazino)carbonyl]-*N*-[(1*S*,2*R*)-1-benzyl-2-hydroxy-3-{[3-(trifluarométhyl)benzyl]amino}propyl]-1-éthyl-1*H*-pyrazole-3-carboxamide ;
- *N*³-[(1*S*,2*R*)-1-benzyl-2-hydroxy-3-{[3-(trifluorométhyl)benzyl]amino}propyl]-1-éthyl-*N*⁵-[2-(méthoxyméthyl)pyrrolidin-1-yl]-1*H*-pyrazole-3,5-dicarbaxamide ;
- 5-[(2-benzyl-2-éthylhydrazino)carbonyl]-*N*-[(1*S*,2*R*)-1-(3,5-difluorobenzyl)-2-hydroxy-3-{[3-(trifluorométhyl)benzyl]amino}propyl]-1-éthyl-1*H*-pyrazole-3-carboxamide ;
- *N*³-[(1*S*,2*R*)-1-(3,5-difluorobenzyl)-2-hydroxy-3-{[3-(trifluorométhyl)benzyl]amino}propyl]-1-éthyl-*N*⁵-[2-(méthoxyméthyl)pyrrolidin-1-yl]-1*H* pyrazole-3,5-dicarboxamide.

Les combinaisons des groupes selon l'invention tels que précédemment définis font également partie de l'invention.

Conformément à l'invention, on peut préparer les composés de formule (Iter) selon le procédé illustré par le schéma 1 qui suit.
Dans les schémas qui suivent, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

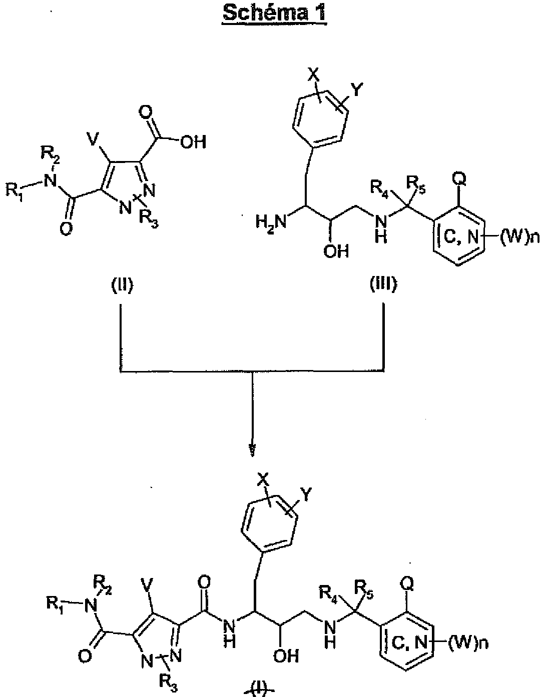

Selon le schéma 1 ci-dessus, le composé de formule (Iter) peut être obtenu par acylation de l'amine de formule (III) - dans laquelle X, Y, R₄, R₅, Q, W et n sont tels que définis pour le composé de formule (Iter) avec l'acide pyrazole de formule (II) - dans laquelle R₁ R₂, R₃ et V sont tels que définis pour le composé de formule (Iter) selon des conditions connues de l'homme du métier, par exemple en présence d'hydrate d'hydroxybenzotriazole (HOBt) et de chlorhydrate de 1-éthyl-3-(3-diméthylaminopropyl)carbodiimide (EDAC) dans un solvant inerte tel que le *N*,*N*'*-*diméthylformamide (DMF), l'acétonitrile ou le dichlorométhane (DCM) à une température pouvant aller de 0°C à la température ambiante (B.M. Trost, I. Fleming, E. Winterfeldt, Comprehensive Organic Synthesis, 1991, vol. 6, 1st edition, Pergamon Press, Oxford, 381-417). Le composé de formule (Iter) est préférentiellement synthétisé à partir de l'amine de formule (III) chirale (2*R*,3*S*) et dans ce cas, la conformation du composé de formule (Iter) final est (1*S*,2*R*).

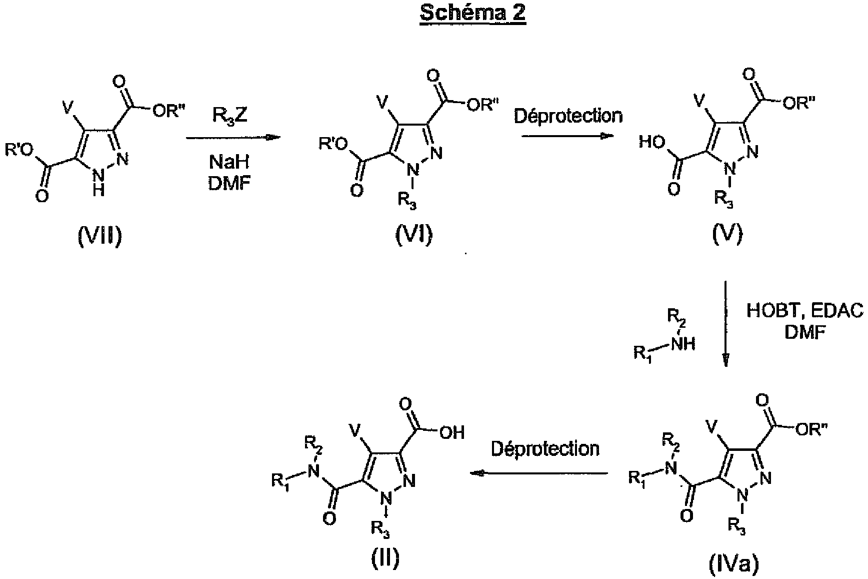

Comme décrit dans le schéma 2 ci-dessus, le composé de formule (II) précédent peut être obtenu par une séquence de réactions partant du pyrazole 3,5-dicarboxylate de dialkyle de formule (VII) dans lequel R' et R" peuvent représenter indépendamment l'un de l'autre un groupe C₁₋₆-alkyle, en particulier un méthyle, un éthyle, un *tert*-butyle, ou un benzyle. Ce pyrazole disubstitué de formule (VII) est soit commercialement disponible tel que le 1*H-*pyrazole-3,5-dicarboxylate de diéthyle, soit synthétisé selon des méthodes connues de l'homme de métier (J.A. Joule, K. Mills, Heterocyclic Chemistry, 2000, 4th edition, Blackwell Science, 448).

Plus précisément, selon le schéma 2, on obtient le dérivé pyrazole *N*-substitué de formule (VI) - dans laquelle R₃ et V sont tels que définis pour le composé de formule (Iter) et R' et R" représentent des groupes protecteurs des fonctions acide carboxylique, par exemple un groupe éthyle (T.W. Greene, P.G.M. Wuts Protective Groups in Organic Synthesis, 1999, 3rd Edition, John Wiley and Sons, Inc, 369-431), par réaction de substitution d'un dérivé diester *NH*-pyrazole de formule (VII) - dans laquelle V, R' et R" sont tels que définis pour le composé de formule (VI) - par un agent alkylant de formule R₃-Z dans laquelle Z est un groupement nucléofuge connu de l'homme du métier comme par exemple un halogénure, un mésylate, un tosylate. Cette réaction est effectuée en présence d'une base telle que l'hydrure de sodium, dans un solvant inerte tel que le DMF (S.C. Kuo, L.J. Huang, H. Nakamura, J. Med. Chem ; 1984, 49, 539).

Dans le cas où l'on veut obtenir des dérivés pyrazole de formule (VI) dans laquelle R₃ est un groupe aryle ou hétéroaryle, on fait par exemple subir aux composés de formule (VII), tels que décrits précédemment, une réaction de substitution nucléophile aromatique ou une réaction du type Ullmann (R. Bambal, R.B. Hazilnik, J. Org. Chem. 1994, 59, 729-732 ; J. Hassan, M. Sevignon, C. Gozzi, E. Schultz, M. Lemaire, Chem. Rev., 2002, 102, 1359-1470; A. Klapars, J.C. Antilla; X. Huang, S.I. Buchwald, J. Am. Chem. Soc., 2001, 123, 7727-7729).

On obtient le composé de formule (V) - dans laquelle R₃, V et R" sont tels que définis pour le composé de formule (VI) - par déprotection sélective en position 5 du composé de formule (VI) obtenu précédemment, selon des méthodes connues de l'homme du métier (T.W. Greene, P.G.M. Wuts Protective Groups in Organic Synthesis, 1999, 3rd Edition, John Wiley and Sons, Inc, 369-431).

Cette réaction de déprotection sélective peut par exemple être effectuée en présence d'hydroxyde de lithium dans un mélange équivolumique de tétrahydrofurane et d'eau distillée à température ambiante, notamment dans le cas où R' et R" sont tous les deux un groupement éthyle.

On obtient le dérivé monoester de formule (IVa) - dans laquelle R₁, R₂, R₃ et V sont tels que définis pour le composé de formule (Iter) et R" est tel que défini pour le composé de formule (V) - par réaction d'acylation du composé de formule R₁R₂NH dans laquelle R₁ et R₂ sont tels que définis pour le composé de formule (Iter) - par un dérivé acide de formule (V), tels que définis précédemment, selon des conditions connues de l'homme du métier, par exemple en présence de HOBt et d'EDAC et dans un solvant inerte tel que le DMF (B.M. Trost, I. Fleming, E. Winterfeldt Comprehensive organic Synthesis, 1991, vol. 6, 1st edition, Pergamon Press, Exeter, 381-417).

On obtient enfin le composé de formule (II) dans laquelle R₁, R₂, R₃ et V sont tels que définis pour le composé de formule (Iter), par réaction de déprotection du composé de formule (IVa) obtenu précédemment, selon des méthodes connues de l'homme du métier, par exemple en présence d'hydroxyde de lithium, dans un mélange équivolumique de THF et d'eau distillée à température ambiante, en particulier lorsque R" représente un groupe éthyle.

Alternativement, on peut obtenir les composés de formule (II) dans lesquels R₃ est un alkyle, à partir du dérivé de formule (IVa) dans laquelle R₃ est un benzyle, les autres groupements étant tels que définis précédemment.

Dans ce cas, le dérivé de formule (IVa) est débenzylé selon les conditions de déprotection connue de l'homme du métier, comme par exemple en présence de formate d'ammonium et de 10 % Pd/C dans l'éthanol absolu (T.W. Greene, P.G.M. Wuts Protective Groups in Organic Synthesis, 1999, 3rd Edition, John Wiley and Sons, Inc, 578-580).

L'amide *N*-débenzylé ainsi obtenu, subit ensuite une réaction d'alkylation suivant les conditions connues de l'homme du métier, telles que celles utilisées pour la synthèse du composé de formule (VI), permettant d'obtenir les 2 régioisomères possibles qui sont ensuite déprotégés en acides.

Alternativement, on peut obtenir les compositions de formule (IVa), permettant d'obtenir le composé de formule (II), selon le schéma 3 qui suit.

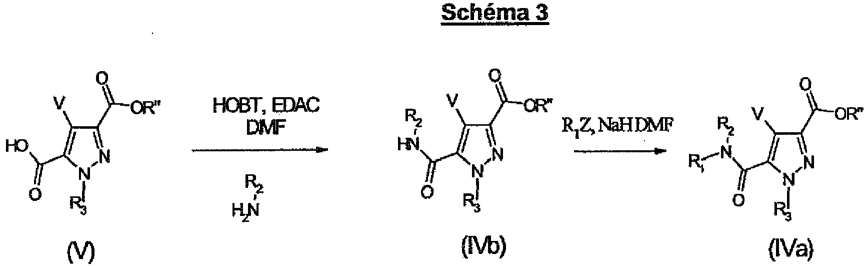

Ainsi, on obtient les composés de formule (IVa) telle que définie précédemment, par réaction de N-alkylation sur le composé de formule (IVb) - dans laquelle R₂, R₃, V et R" sont tels que définis pour le composé de formule (IVa)- d'après les méthodes connues de l'homme du métier (R.C. Larock Comprehsensive Organic Transformations, 1999, 2nd Edition, John Wiley & Sons, Inc., New York, 1979-1980), par un agent alkylant par exemple de formule R₁-Z dans laquelle R₁ est tel que défini pour le composé de formule (Iter) (à l'exception d'un hydrogène) et Z est tel que défini précédemment.

On obtient le composé de formule (lVb) par réaction d'acylation du composé de formule R₂-NH₂ par le dérivé acide de formule (V) tel que défini précédemment, selon les conditions connues de l'homme du métier, par exemple en présence de HOBt et d'EDAC dans un solvant inerte tel que du DMF (B.M. Trost, I. Fleming, E. Winterfeldt Comprehensive organic Synthesis, 1991, vol. 6, 1st edition, Pergamon Press, Exeter, 381-417).

Les dérivés amines de formule (III) peuvent être obtenus selon le schéma 4 qui suit.

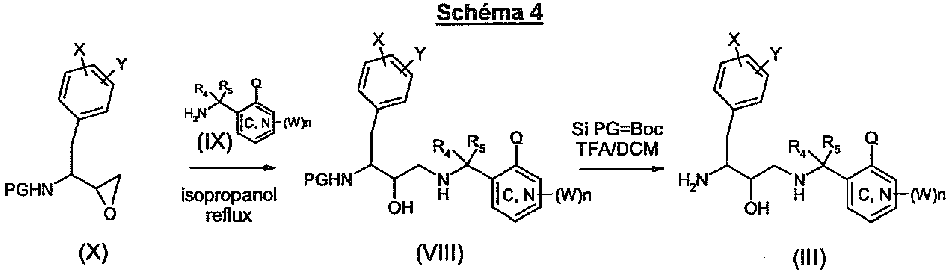

On obtient les composés de formule (III) - dans laquelle R₄, R₅, X, Y, Q, W et n sont tels que définis pour le composé de formule (Iter) -, par réaction de déprotection du dérivé amine de formule (VIII) - dans laquelle R₄, R₅, X, Y, Q, W et n sont tels que définis pour le composé de formule (Iter) et PG représente un groupe protecteur (T.W. Greene, P.G.M. Wuts Protective Groups in Organic Synthesis, 1999, 3rd Edition, John Wiley and Sons, Inc, 369-431).

Par exemple, si le groupe protecteur est un *tert*-butoxycarbonyl (Boc), le dérivé amine de formule (VlII) est traité par de l'acide trifluoroacétique dans du dichlorométhane (mélange équivolumique).

On obtient le composé de formule (VIII) tel que défini précédemment, par réaction du composé de formule (X) - dans laquelle X et Y sont tels que définis pour le composé de formule (Iter) et PG représente un groupe protecteur - avec un composé de formule (IX) - dans laquelle R4, R5, Q, W et n sont tels que définis pour le composé de formule (Iter) selon les conditions connues de l'homme du métier, par exemple dans l'isopropanol à reflux (A.K. Gosh, S. Leshchenko, M. Noetzel J. Org. Chem., 2004, 69, 7822-7829) ou dans le dichloroéthane (DCE) chauffé entre 40 °C et 85 °C en présence d'une quantité catalytique de trifluorométhane sulfonimide de lithium (J. Cossy, V. Bellosta, C. Hamoir, J.-R. Desmurs Tetrahedron Lett., 2002,43, 7083-7086).

Le composé de formule (IX) est soit commercialement disponible soit synthétisé à partir de composés commerciaux et suivant des méthodes connues de l'homme du métier (E. Ciganek J. Org. Chem., 1992, 57, 4521-4527; P. Bertus, J. Szymoniak, J. Org. Chem., 2003, 68, 7133-7136; T.T. Colyer, N.G. Andersen, J.S. Tedrow, T.S. Soukup, M.M. Faul, J. Org. Chem., 2006, 71, 6859-6862).

Le composé de formule (X) est soit commercialement disponible soit synthétisé suivant des méthodes connues de l'homme du métier (R. Luly, J.F. Dellaria, J.J. Plattner, J.L. Soderquist, N. Yi, J. Org. Chem., 1987, 52,1487).

De préférence, le dérivé amine de formule (III) sous forme énantiomérique (*S*,*R*) est obtenu à partir du composé de formule (X) chiral (S,S).

Lorsqu'une fonction d'un composé est réactive, par exemple lorsque R₁ comporte un hydroxy, elle peut nécessiter une protection préalable avant réaction. L'homme du métier pourra déterminer aisément la nécessité d'une protection préalable (T.W. Greene, P.G.M. Wuts Protective Groups in Organic Synthesis, 1999, 3rd Edition, John Wiley and Sons, Inc, 369-431).

Dans les préparations sont décrites les méthodes de synthèse des différents intermédiaires permettant d'obtenir les composés de l'invention. Lorsque ces synthèses ne sont pas décrites, les intermédiaires sont soit connus, soit préparés selon des méthodes bien connues de l'homme du métier.

Les abréviations et symboles utilisés pour la description des modes opératoires de synthèse et pour la description des composés sont les suivants:
- DMF pour diméthylformamide,
- DMSO pour diméthylsulfoxyde,
- THF pour tétrahydrofurane,
- AcOEt pour acétate d'éthyle
- CH₃CN pour acétonitrile
- Et₂O pour éther de diéthyle
- MeOH pour méthanol
- DCM pour dichlorométhane
- HCl pour acide chlorhydrique,
- LiOH pour hydroxyde de lithium,
- TFA pour acide trifluoroacétique
- HOBT pour hydrate d'hydroxybenzotriazole
- EDAC pour chlorhydrate de 1-éthyl-3-(3-diméthylaminopropyl)carbodiimide
- NaH pour hydrure de sodium
- 10% Pd/C pour 10% de palladium sur charbon
- Boc pour *tert-*butoxycarbonyl
- Et pour éthyle,
- Me pour méthyle.

Les composés chiraux 34 et 35 sont obtenus par HPLC (High Pressure Liquid Chromatography) préparative chirale du composé 33. La séparation est réalisée sur une HPLC waters Prep 4000 avec une phase stationnaire chirale, la Chiralcel OD-CSP et les composés sont élués avec un mélange d'isohexane et d'isopropanol (95/5, v/v) contenant 0.5% d'isopropylamine à 25°C. La détection est faite à 230nm et le débit utilisé est de 120ml/min. La pureté isomérique de chaque composé chiral est déterminée sur une SFC Berger analytique avec une phase stationnaire Chiralcel OD-H (250x4,6mm, 5µm) et comme éluant un mélange de dioxyde de carbone et d'isopropanol (87/13) contenant 0.5% d'isopropylamine. La stéréochimie exacte au niveau de l'amide coniine des composés 34 et 35 n'a pas été déterminée.

Les spectres de résonance magnétique nucléaire du proton (RMN ¹H) ont été effectués à 300MHz, 500 MHz ou 600 MHz sur des appareils Brüker. Les abréviations utilisées pour caractériser les signaux sont les suivantes: s = singulet, bs = singulet large, m= multiplet, d = doublet, dd=doublet de doublet, t = triplet, q = quadruplet.

La LC-MS (Liquid Chromatography/Mass Spectrometry) est réalisée avec un sytème LC-MS ZMD de Waters équipé d'un Alliance 2695 et d'un détecteur UV à barrette de diodes 996 (200 à 400nm) de Waters piloté avec le logiciel MassLynx V4.1 à 40°C. La colonne utilisée est une Kromasil C18 (50x2mm, 3,5µm, 100Ǻ AIT) et un mélange d'eau distillée contenant 0.05% TFA et d'acétonitrile contenant 0.035%TFA (2% à 100% CH₃CN + 0,035%TFA en 10 min).

La quantification des sels et des solvats est déterminée à l'aide de l'analyse élémentaire, du dosage de l'eau par la technique de Karl-Fischer et de l'intégration des signaux caractéristiques des solvants en RMN¹H.

### Synthèse des intermédiaires de formules (II) et (III)

### Intermédiaire 1 de formule (II): synthèse de l'acide 1-éthyl-5-[(2-propylpiperidin-1-yl)carbonyl]-1H-pyrazole -3-carboxylique

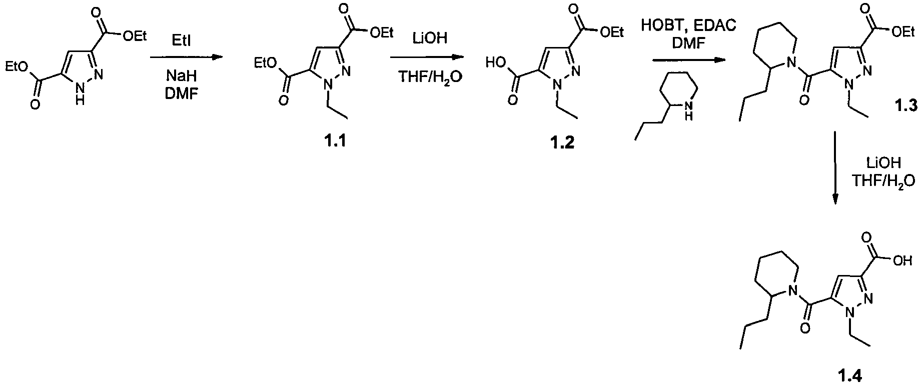

### 1.1/ 1-éthvl-1H-pyrazole-3,5-dicarboxylate de diéthyle

Après avoir lavé 3,11g d'hydrure de sodium avec du pentane, on ajoute 5ml de DMF. Le 1*H*-pyrazole-3,5-dicarboxylate de diéthyle, préalablement dissous dans 120ml de DMF, est ajouté goutte à goutte à température ambiante. Le mélange réactionnel ainsi obtenu est laissé sous agitation durant 2h30 à cette température et puis l'iodure d'éthyle, préalablement dissous dans 60ml de DMF, est ajouté. Après addition de l'iodure d'éthyle, l'agitation est maintenue durant 15h, puis le mélange réactionnel est évaporé à sec. Le résidu obtenu est repris dans l'AcOEt et la phase organique est alors lavée 2 fois à l'eau distillée, séchée sur du sulfate de sodium anhydre et concentrée sous vide. Le résidu obtenu est chromatographié sur une colonne de gel de silice en éluant avec un mélange d'AcOEt et d'éther de pétrole 1/9 (v/v) et le produit du titre est obtenu sous forme d'huile jaune (14,40g).
¹H RMN (300MHz, CDCl₃) δppm : 1,40 (m, 6H), 4,36 (q, 4H), 4,65 (q, 2H), 7,31 (s, 1H).

### 1.2/ acide 3-(éthoxycarbonyl)-1-éthvl-1H-pyrazole-5-carboxylique

A une solution du diester **1.1** (7,46g) précédemment obtenue dans 350ml de mélange de THF et d'eau distillée 1/1 (v/v) à température ambiante, on ajoute 1,24g d'hydroxyde de lithium. Au bout d'1 h d'agitation, le pH est ramené à 2 et le mélange réactionnel est extrait avec de l'AcOEt. Les phases organiques combinées sont lavées avec de l'eau distillée, puis séchées sur du sulfate de sodium anhydre et concentrées sous vide. Le résidu obtenu est chromatographié sur une colonne de gel de silice en éluant avec un gradient de DCM et d'isopropanol contenant 10% en volume d'acide acétique (gradient de 0 à 5% isopropanol + 10%AcOH) et le produit du titre est isolé sous forme d'un solide blanc (4,69g).
¹H RMN (300MHz, CDCl₃) δppm : 1,42 (t, 3H), 1,51 (t 3H), 4,45 (q, 2H), 4,72 (q, 2H), 7,50 (s, 1 H), 10,15 (bs, 1 H).

### 1.3 / 1-éthyl-5-[(2-propylpiperidin-1-yl)carbonyl]-1H-pyrazole-3-carboxylate d'éthyle

A une solution de monoacide **1.2** (7,00g) précédemment obtenu dans 140ml de DMF à température ambiante, on ajoute 5,56g d'hydrate d'HOBt et 6,96g d'EDAC, et la solution obtenue est laissée sous agitation durant 10 min. Ensuite 3,99g de coniine racémique sont ajoutés et le mélange réactionnel est laissé sous agitation pendant 15h. Le solvant est évaporé à sec et le résidu est repris dans de l'AcOEt, puis est lavé 2 fois avec une solution saturée de bicarbonate de sodium et 1 fois avec de l'eau distillée. La phase organique est séchée sur du sulfate de sodium anhydre et concentrée sous vide. Le résidu obtenu est chromatographié sur une colonne de gel de silice en éluant avec un gradient d'éther de pétrole et d'AcOEt (gradient de 0 à 20% AcOEt) et le produit du titre est obtenu sous forme d'une huile incolore (5,63g).
¹H RMN (300MHz, CDCl₃) δppm : 0,7-0,9 (m, 3H), 1,05-1,9 (m 16H), 2,8-3,2 (m, 1 H), 3,6-4,1 (m, 1 H), 4,35 (m, 4H), 4,40-4,90 (m, 1 H), 6,74 (s, 1 H).

### 1.4 / acide 1-éthyl-5-[(2-propylpiperidin-1-yl)carbonyl]-1H-pyrazole-3-carboxylique

A une solution de l'ester **1.3** (2,21g) précédemment obtenu dans 50ml de mélange de THF et d'eau distillée 1/1 (v/v) à température ambiante, on ajoute 0,57g d'hydroxyde de lithium. Au bout de 4h30 d'agitation, le solvant est évaporé et le résidu est repris dans l'eau distillée et l'AcOEt. Le pH est ramené à 2 et puis le mélange réactionnel est extrait avec de l'AcOEt. Les phases organiques combinées sont lavées avec de l'eau distillée, séchées sur du sulfate de sodium anhydre et concentrées sous vide : le produit du titre est isolé sous forme d'une huile incolore (1,91g).
¹H RMN (300MHz, CDCl₃) δppm : 0,75-1,05 (m, 3H), 1,05-1,9 (m 13H), 2,7-3,2 (m, 1 H), 3,6-4,1 (m, 1 H), 4,35 (m, 2H), 4,45-4,95 (m, 1 H), 6,80 (s, 1 H).

### Intermédiaire 2 de formule (II) : synthèse de l'acide 5-(dipropylcarbamoyl)-1-éthyl-1H-pyrazole-3-carboxylique

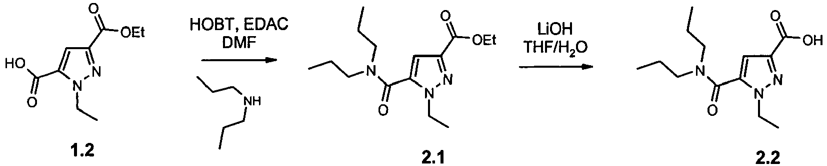

### 2.1/ 5-(dipropylcarbamoyl)-1-éthyl-1H-pyrazole-3-carboxylate d'éthyle

A une solution de monoacide **1.2** (15,00g) dans 300ml de DMF à température ambiante, on ajoute 11,04g d'HOBt et 13,64g d'EDAC et la solution obtenue est laissée sous agitation durant 10 min. Ensuite 2,30g de dipropylamine sont ajoutés et le mélange réactionnel est laissé sous agitation pendant 15h. Le solvant est évaporé à sec et le résidu est repris dans l'AcOEt, puis lavé 2 fois avec une solution saturée de bicarbonate de sodium et 1 fois avec de l'eau distillée. La phase organique est séchée sur du sulfate de sodium anhydre et concentrée sous vide. le produit du titre est isolé sous forme d'une huile incolore (19,00g).
LCMS : MH⁺= 296

### 2.2/ acide 5-(dipropylcarbamovl)-1-éthyl-1H-pyrazole-3-carboxylique

A une solution d'ester **2.1** (7,00g) dans 300ml de mélange de THF et d'eau distillée 7/3 (v/v) à température ambiante, on ajoute 1,52g d'hydroxyde de lithium et le mélange réactionnel est chauffé à 60°C pendant 1h30. Le pH est ramené à 2 et le mélange réactionnel est extrait avec de l'AcOEt. Les phases organiques combinées sont séchées sur du sulfate de sodium anhydre et concentrées sous vide : le produit du titre est obtenu sous forme d'un solide blanc (5,2g).
LCMS : MH⁺= 268

### Intermédiaire 3 de formule (II) : synthèse de l'acide 5-[(2-benzyl-2-éthylhydrazino)carbonyl]-1-éthyl-1H-pyrazole-3-carboxylique

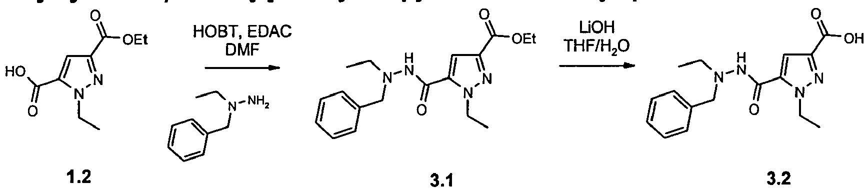

### 3.1 / 5-[(2-benzyl-2-éthylhydrazino)carbonyl]-1-éthyl-1H-pyrazole-3-carboxylate d'éthyle

A une solution de monoacide **1.2** (1,00g) dans 20ml de DMF à température ambiante, on ajoute 0,79g d'HOBt et 0,99g d'EDAC et la solution obtenue est laissée sous agitation durant 10 min. Ensuite 0,78g de *N*-benzyl-*N*-éthyl-hydrazine sont ajoutés et le mélange réactionnel est laissé sous agitation pendant 15h. Le solvant est évaporé à sec et le résidu est repris dans de l'AcOEt, puis lavé 2 fois avec une solution saturée de bicarbonate de sodium et 1 fois avec de l'eau distillée. La phase organique est séchée sur du sulfate de sodium anhydre et concentrée sous vide. Le résidu obtenu est chromatographié sur une colonne de gel de silice en éluant avec un gradient d'éther de pétrole et de l'AcOEt (gradient de 0 à 25% AcOEt) et le produit du titre est obtenu sous forme d'une huile incolore (1,53g).
¹H RMN (300MHz, CDCl₃) δppm : 0,8 (m, 3H), 1,08-1,38 (m, 6H), 2,91 (m, 2H), 4,03 (m, 2H), 4,32 (m, 2H), 4,48 (m, 2H), 6,80 (s, 1 H), 7,25 (m, 5H).

### 3.2/ acide 5-[(2-benzyl-2-éthylhydrazino)carbonyl]-1-éthyl-1H-pyrazole-3-carboxylique

A une solution d'ester **3.1** (1,53g) dans 40ml de mélange de THF et d'eau distillée 1/1 (v/v) à température ambiante, on ajoute 0,37g de LiOH. Au bout de 3h30 sous agitation, le pH est ramené à une valeur allant de 6 à 7 et le mélange réactionnel est extrait avec de l'AcOEt. Les phases organiques combinées sont lavées avec de l'eau distillée, séchées sur du sulfate de sodium anhydre et concentrées sous vide : le produit du titre est obtenu sous forme d'un solide blanc (0,59g).
¹H RMN (300MHz, CDCl₃) δppm : 0,82 (m, 3H), 1,45 (t, 3H), 3,39 (m, 2H), 4,37 (m, 2H), 4,69 (q, 2H), 7,09 (s, 1 H), 7,22 (m, 5H).

### Intermédiaire 4 de formule (III) : synthèse du (2R,3S)-3-amino-4-phényl-1-{[3-(trifluorométhyl) benzyl]-amino}butan-2-ol

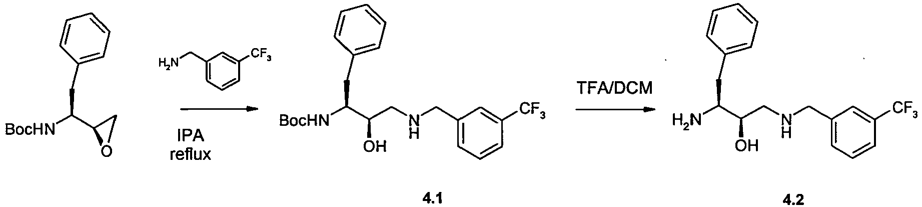

### 4.1/ [(1S,2R)-1-benzyl-2-hydroxy-3-{[3-(trifluorométhyl)benzyl]amino} propyl] carbamate de tert-butyle

Dans 151ml d'isopropanol à température ambiante, on dissous 3,97g de l'époxyde (S,S) et 3,43g de 3-trifluorométhylbenzylamine. Le mélange réactionnel est ensuite chauffé à reflux durant 15h. Une fois à température ambiante, un précipité s'est formé et est donc filtré : le produit du titre est ainsi isolé sous forme d'un solide blanc (2,69g).
LCMS : MH⁺= 439

### 4.2/ (2R,3S)-3-amino-4-phényl-1-{[3-(trifluorométhyl)benzyl]amino}butan-2-ol

Dans 7ml de DCM, 2,50g de l'amine protégée **4.1** précédemment obtenus, sont dissous et on ajoute 7ml de TFA. Le mélange réactionnel est laissé sous agitation durant 1 h à température ambiante, puis basifié avec une solution saturée de bicarbonate de sodium. Ce mélange biphasique est extrait avec du DCM, et la phase organique est séchée sur du sulfate de sodium anhydre et concentrée sous vide. Le résidu obtenu est chromatographié sur une colonne de gel de silice en éluant avec un gradient de DCM et de méthanol (gradient de 0 à 5% MeOH) et le produit du titre est obtenu sous forme d'une huile incolore (1,74g).
LCMS : MH⁺= 339

### Intermédiaire 5 de formule (III) : synthèse du (2R,3S)-3-amino-4-(3,5-difluorophényl)-1-{[3-(trifluorométhyl)benzyl]amino}butan-2-ol

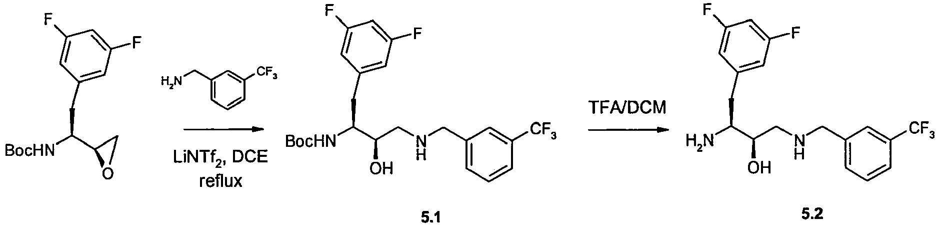

### 5.1/ [(1S,2R)-1-(3,5-difluorobenzyl)-2-hydroxy-3-{[3-(trifluorométhyl) benzyl]amino} propyl] carbamate de tert-butyle

Dans 50 ml de dichloroéthane (DCE) à température ambiante, on dissout 2,00g de l'époxyde (S,S) et 1,41g de 3-trifluorométhylbenzylamine, puis on ajoute 0,19g de trifluorométhanesulfonimide de lithium. Le mélange réactionnel est ensuite chauffé à 50°C durant 64h. Une fois à température ambiante, la solution est lavée avec de l'eau distillée, et la phase organique est séchée sur du sulfate de sodium anhydre et concentrée sous vide. Le résidu obtenu est chromatographié sur une colonne de gel de silice en éluant avec un gradient de DCM et de méthanol (gradient de 0 à 5% MeOH) et le produit du titre est obtenu sous forme d'une huile incolore (2,39g).
RMN (300MHz, CDCl₃) δppm : 1,38 (s, 9H), 1,80 (bs, 1 H), 2,79 (m, 3H), 3,00 (dd, 1H), 3,49 (m, 1 H), 3,87 (m, 3H), 4,61 (d, 1H), 6,71 (m, 3H), 7,52 (m, 4H).

### 5.2 / (2R,3S)-3-amino-4-(3,5-difluorophényl)-1-{[3-(trifluorométhyl)-benzyl]amino} butan-2-ol

Dans 15ml de DCM, 2,39g de l'amine protégée **5.1** précédemment obtenu sont dissous et on ajoute 15ml de TFA. Le mélange réactionnel est laissé sous agitation durant 1 h à température ambiante, puis évaporé à sec. Le résidu est repris avec l'AcOEt et la solution obtenue est lavée avec une solution saturée de carbonate de sodium, et ensuite avec de l'eau distillée. La phase organique est séchée sur du sulfate de sodium anhydre et concentrée sous vide. Le résidu obtenu est chromatographié sur une colonne de gel de silice en éluant avec un gradient de DCM et de méthanol contenant 10% d'hydroxide d'ammonium en volume (gradient de 0 à 5% MeOH+10%NH4OH) et le produit du titre est obtenu sous forme d'une huile incolore (1,66g).
RMN (300MHz, CDCl₃) δppm :2,51 (m, 1H), 2,77 (m, 1 H), 2,92 (m, 2H), 3,13 (m, 1 H), 3,60 (m, 1 H), 3,91 (d, 2H), 6,73 (m, 3H), 7,53 (m, 4H).

### Intermédiaire 6 de formule (III) : synthèse du (2R,3S)-3-amino-4-(3,5-difluorophényl)-1-{[(1S)-5-(trifluorométhyl)-1,2,3,4-tétrahydronaphtalèn-1-yl]amino}butan-2-ol

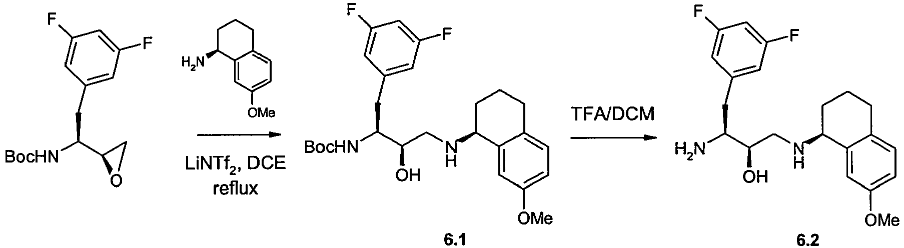

### 6.1/ [(1S,2R)-1-(3,5-difluorobenzyl)-2-hydroxy-3-{[(1S)-5-(trifluorométhyl)-1,2,3,4-tétrahydronaphtalèn-1-yl]amino}propyl]carbamate de tert-butyle

Dans 10 ml de DCE à température ambiante, on dissout 0,40g de l'époxyde (S,S) et 0,34g de chlorhydrate de la 7-méthoxy-1,2,3,4-tétrahydronaphtylamine, puis on ajoute 0,04g de LiNTf₂. Le mélange réactionnel est laissé sous agitation à température ambiante durant 15h et ensuite chauffé à 50°C durant 39h30. Le mélange réactionnel est concentré à sec, et le résidu est repris dans de l'AcOEt, puis lavé avec de l'eau distillée. La phase organique est séchée sur du sulfate de sodium anhydre et concentrée sous vide. Le résidu obtenu est chromatographié sur une colonne de gel de silice en éluant avec un gradient de DCM et de méthanol (gradient de 0 à 3% MeOH) et le produit du titre est obtenu sous forme d'un solide blanc (0,49g).
LCMS : MH⁺= 477

### 6.2/ (2R,3S)-3-amino-4-(3,5-difluorophényl)-1-{[(1S)-5-(trifluorométhyl)-1,2,3.4-tétrahydronaphtalèn-1-yl]amino}butan-2-ol

Dans 10ml de DCM, on dissout 2,15g de l'amine protégée **6.1** précédemment obtenu et on ajoute 10ml de TFA. Le mélange réactionnel est laissé sous agitation durant 40min à température ambiante, puis évaporé à sec. Le résidu est repris avec l'AcOEt et la solution obtenue est lavée avec une solution saturée de carbonate de sodium, et ensuite avec de l'eau distillée. La phase organique est séchée sur du sulfate de sodium anhydre et concentrée sous vide. Le résidu obtenu est chromatographié sur une colonne de gel de silice en éluant avec un gradient de DCM et de méthanol contenant 10% d'hydroxyde d'ammonium en volume (gradient de 0 à 6% MeOH+10%NH₄OH et le produit du titre est obtenu sous forme d'une huile incolore (1,44g).
RMN (300MHz, CDCl₃) δppm : 1,92 (m, 3H), 2,17 (m, 3H), 2,52 (m, 1H), 2,73 (m, 1H), 2,84 (m, 1H), 2,95-3,15 (m, 3H), 3,56 (m, 1H), 3,82 (s, 3H), 6,67-6,80 (m, 4H), 6,94 (d, 1H), 7,04 (d, 1 H)

Les exemples suivants décrivent la préparation de certains composés conformes à l'invention. Ces exemples ne sont pas limitatifs et ne font qu'illustrer l'invention.

Les numéros des composés exemplifiés renvoient à ceux donnés dans le tableau ci-après. Les micro-analyses élémentaires et les analyses RMN, IR ou LC-MS (chromatographie liquide couplée à la spectrométrie de masse) confirment les structures des composés obtenus.

### Exemples de préparation de composés de formule générale (Iter)

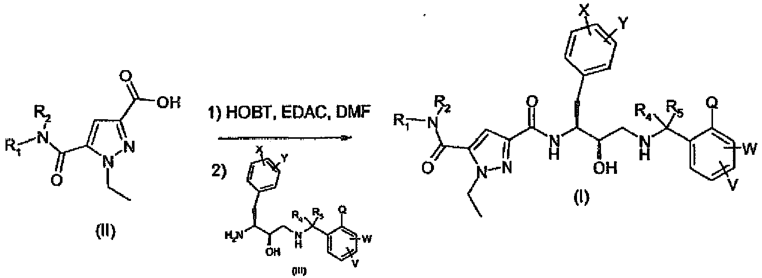

### Exemple 1 : N³[(1S,2R)-1-(3,5-difluorobenzyl)-2-hydroxy-3-{[3-(triftuorométhyl)-benzyl]amino}propyl]-1-éthyl-N⁵-N⁵-dipropyl-1H-pyrazole-3,5-dicarboxamide (composé 5, tableau I)

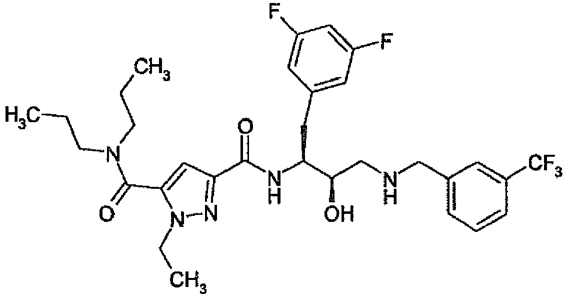

A une solution de 0,15g de l'acide de formule (II) **2.2** dans 8ml de DMF à température ambiante, 0,09g d'HOBt et 0,12g d'EDAC sont ajoutés. La solution obtenue est laissée sous agitation durant 10 min et 0,23g de l'amine de formule (III) **5.2** sont ensuite ajoutés. Le mélange réactionnel est laissé sous agitation durant 72h. Le solvant est évaporé à sec et le résidu est repris dans de l'AcOEt, puis lavé 2 fois avec une solution saturée de bicarbonate de sodium et 1 fois avec de l'eau distillée, La phase organique est séchée sur du sulfate de sodium anhydre et concentrée sous vide. Le résidu obtenu est chromatographié sur une colonne de gel de silice en éluant avec un gradient de DCM et de méthanol (0 à 5% MeOH) et l'aminoalcool est isolé sous forme de base. Après dissolution dans de l'éther de diéthyle (Et₂O) une solution d'acide chlorhydrique 1 N dans de l'Et₂O est ajoutée afin de former le chlorhydrate de l'amine. Ce sel est finalement recristallisé dans un mélange de DCM, d'Et₂O et de pentane, et 0,26g du chlorhydrate du produit 1 sont ainsi isolés.
LCMS : MH⁺=624
¹H RMN (600MHz, DMSO-d₆) δppm : voir Tableau I.

### Exemple 2: 5-[(2-benzyl-2-éthylhydrazino)carbonyl]-N-[(1S,2R)-1-benzyl -2-hydroxy-3-{[3-(trifluorométhyl)benzyl]amino}propyl]-1-éthyl-1H-pyrazole-3-carboxamide (composé 69, Tableau II)

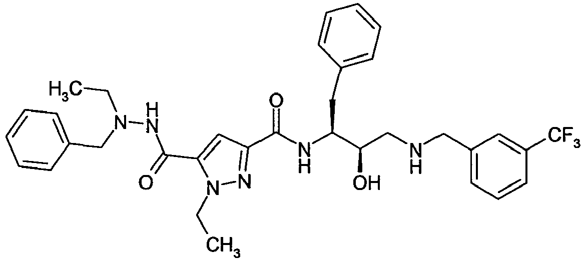

En suivant la même procédure que pour le produit 1 et en utilisant 0.18g de l'acide de formule (II) **3.2,** 0,10g d'HOBt, 0,12g d'EDAC et 0,21g d'amine de formule (III) **4.2** dans 7ml de DMF, 0,23g du chlorhydrate du produit 2 sont isolés après chromatographie avec un gradient de 0 à 4% de MeOH dans DCM suivi de salification,.
LCMS : MH⁺=637
¹H RMN (500MHz, DMSO-d₆) δppm : voir Tableau II

### Exemple 3 : N-[(1S,2R)-1-(3,5-difluorobenzyl)-2-hydroxy-3-{[(1S)-7-(trifluorométhyl)-1,2,3,4-tétrahydronaphtalèn-1-yl]amino}propyl]-1-éthyl-5-[(2-propylpiperidin-1-yl)carbonyl]-1H-pyrazole-3-carboxamide (composé 64, Tableau I)

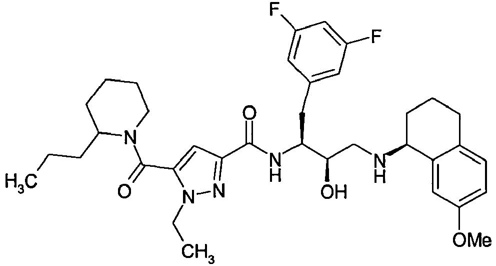

En suivant la même procédure que pour le produit 1 et en utilisant 0,13g de l'acide de formulation (II) **1.4,** 0,07g d'HOBt, 0,09g d'EDAC et 0,18g de l'amine de formule (III) **6.2** dans 7ml de DMF, 0,17g du chlorhydrate du produit 3 sont isolés après chromatographie avec un gradient de 0 à 3% de MeOH dans DCM suivi de salification.
LCMS : MH⁺=652
¹H RMN (500MHz, DMSO-d₆) ôppm : voir Tableau I.

Dans le Tableau I qui suit :
- Me = méthyle,
- Ph = phényle,

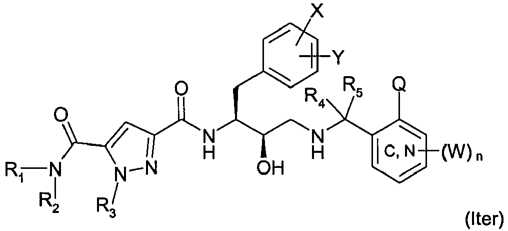

**Tableau I**

| | NR₁R₂ | R₃ | X | Y | 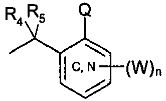 | ¹H RMN δppm (500MHz, DMSO-d₆) **Si 600MHz et DMSO-d₆ |
|---|---|---|---|---|---|---|
| 1 | 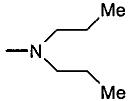 | Et | H | H | 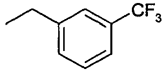 | 0,75 (m, 3H), 0,91 (m, 3H), 1,36 (m, 3H), 1,52 (m, 2H), 2,90 (m, 2H), 3,13 (m, 2H), 3,23 (m, 2H), 3,39 (m, 2H), 3,92 (m, 1H), 4,13 (m, 1H), 4,17 (m, 2H), 4,32 (m, 2H), 5,86 (bs, 1 H), 6,68 (s, 1 H), 7,14 (m, 1 H), 7,23 (m, 4H), 7,64 (t, 1 H), 7,76 (d, 1 H), 7,82 (d, 1 H), 7,95 (s, 1 H), 8,14 (d, 1H), 9,09 (bs, 2H) |
| 2 | 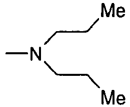 | Et | H | H | 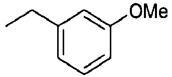 | 0,49 (m, 3H), 0,65 (m, 3H), 1,09 (t, 3H), 1,27 (m, 2H), 1,35 (m, 2H), 2,59 (m, 1 H), 2,63 (d, 1H), 2,70 (d, 1 H), 2,87 (m, 1 H), 2.97 (m, 2H) 3.14 (m, 2H), 3.50 (s, 3H), 3.66 (m, 1 H), 3,91 (m, 5H), 5,58 (bs, 1 H), 6,42 (s, 1 H), 6,68 (m, 1H), 6,79 (m, 2H), 6,94 (m, 2H), 6,97 (m, 3H), 7,06 (m, 2H), |
| | | | | | | 7,85 (d, 1 H), 8,68 (bs, 2H) |
| 3 | 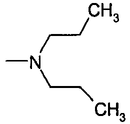 | Et | prop-2-èn-1-yloxy | H | 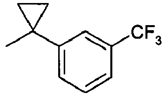 | 0,81 (m, 3H), 0,98 (m, 3H), 1,28 (m, 1H), 1,41 (m, 4H), 1,64 (m, 6H), 2,80 (m, 1 H), 2,90 (t, 1 H), 3,07 (m, 1 H), 3,29 (m, 2H), 3,45 (m, 2H), 3,97 (m,1 H), 4,10 (m, 1H), 4,23 (m, 2H), 4,51 (m, 2H), 5,27 (d, 1 H), 5,37 (d, 1 H), 5,89 (d, 1 H), 6,04 (m, 1 H), 6,70 (s, 1 H), 6,76 (d, 1 H), 6,78 (s, 1 H), 6,82 (s, 1 H), 7,17 (t, 1H), 7,63 (m, 1H), 7,78 (m, 1H), 7,80 (m, 1H), 8,01 (s, 1 H), 8,13 (d, 1 H), 9,47 (bs, 1H), 9,60 (bs,1H) |
| 4 | 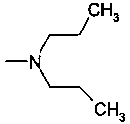 | Et | H | benzyl-4-oxy | 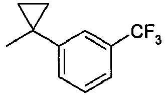 | 0,63 (m, 3H), 0,79 (m, 3H), 1,10 (m, 1H), 1,23 (m, 4H), 1,45 (m, 6H), 2,66 (m, 2H), 2,79 (m, 2H), 3,11 (m, 2H), 3,28 (m, 2H), 3,76 (m, 1H), 3,89 (m,1 H), 4,05 (m, 2H), 5,69 (d, 1 H), 6,25 (s, 1H), 6,70 (d, 2H), 6,99 (d, 2H), 7,20 (m, 1H), 7,27 (m, 4H), 7,45 (m, 1H), 7,59 (m, 1H), 7,71 (m, 1H), 7,83 (s, 1 H), 7,92 (d, 1 H), 9,30 (bs, 1H), 9,47 (bs, 1H) |
| 5 | 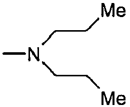 | Et | 3-F | 5-F | 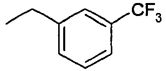 | ** 0,72 (m, 3H), 0,89 (m, 3H), 1,33 (t, 3H), 1,49 (m, 2H), 1,58 (m, 2H), 2,89 (m, 2H), 3,12 (m, 2H), 3,20 (m, 2H), 3,37 (m, 2H), 3,92 (m, 1H), 4,18 (m, |
| | | | | | | 2H), 4,28 (m, 2H), 5,87 (d, 1 H), 6,67 (s, 1 H), 6,95 (m, 3H), 7,63 (t, 1H), 7,74 (d, 1H), 7,82 (d, 1H), 7,94 (s, 1H), 8,155 (d, 1H), 8,98 (bs, 1H), 9,16 (bs, 1H) |
| 6 | 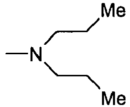 | 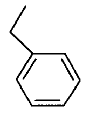 | H | H | 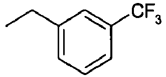 | 0,59 (m, 3H), 0,82 (m, 3H), 1,25 (m, 2H), 1,48 (m, 2H), 2,87 (m, 2H), 2,99 (m, 2H), 3,11 (m, 2H), 3,27 (m, 2H), 3,95 (m, 1H), 4,08 (m, 1 H), 4,27 (m, 2H), 5,40 (s, 2H), 5,90 (d, 1H), 6,73 (s, 1H), 7,17 (m, 7H), 7,33 (m, 3H), 7,62 (t, 1H), 7,74 (d, 1H), 7,81 (d, 1H), 7,95 (s, 1H), 8,16 (d, 1H), 9,03 (bs, |
| | | | | | | 1H), 9,24 (bs, 1 H) |
| 7 | 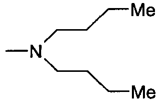 | Et | H | H | 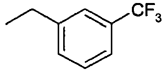 | ^{**}0,77 (m, 3H), 0,91 (m, 3H), 1,13 (m, 2H), 1,31 (m, 5H), 1,45 (m, 2H), 1,54 (m, 2H), 2,87 (m, 2H), 3,08 (m, 2H), 3,24 (m, 2H), 3,39 (m, 2H), 3,87 (m, 1 H), 4,12 (m, 3H), 4,27 (m, 2H), 5,85 (bs, 1 H), 6,65 (s, 1 H), 7,11 (m, 1H), 7,20 (m, 4H), 7,61 (t, 1H), 7,73 (d, 1H), 7,80 (d, 1H), 7,93 (s, 1H), 8,06 |
| | | | | | | (d, 1H), 8,98 (bs, 1H), 9,16 (bs, 1H) |
| 8 | 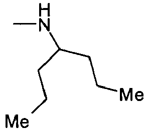 | Et | H | H | 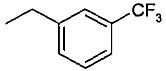 | 0,83 (m, 6H), 1,27 (m, 7H), 1,40 (m, 4H), 2,85 (m, 2H), 3 (d, 1 H), 3,17 (d, 1 H), 3,88 (m, 2H), 4,09 (m, 1 H), 4,22 (m, 2H), 4,48 (m, 2H), 5,83 (bs, 1 H), 7,11 (m, 2H), 7,20 (m, 4H), 7,60 (t, 1 H), 7,70 (d, 1 H), 7,77 (d, 1 H), 7,89 (s, 1 H), 8,08 (d, 1 H), 8,13 (d, 1H), 9,00 (bs, 2H) |
| 9 | 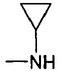 | Et | H | H | 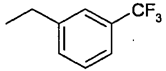 | ^{**} 0,54 (m, 2H), 0,68 (m, 2H), 1,33 (t, 3H), 2,84 (m, 3H), 3,07 (m, 2H), 3,93 (m, 1 H), 4,11 (m, 1 H), 4,25 (m, 2H), 4,52 (m, 2H), 5,85 (bs, 1 H), 7,11 (m, 2H), 7,19 (m, 4H), 7,62 (t, 1H), 7,73 (d, 1H), 7,80 (d, 1 H), 7,93 (s, 1H), 8,05 (d, 1 H), 8,50 (s, 1H), 8,99 (bs, 1H), 9,19 (bs, 1 H) |
| 10 | 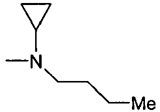 | Et | H | H | 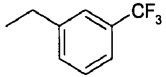 | 0,48 m, 2H), 0,63 (m, 2H), 0,91 (m, 3H), 1,33 (m, 5H), 1,58 (m, 2H), 2,88 (m, 3H), 3,11 (m, 2H), 3,43 (m, 2H), 3,94 (m, 1 H), 4,09 (m, 1 H), 4,25 (m, 4H), 5,89 (d, 1 H), 6,90 (m, 1 H), 7,12 (m, 1 H), 7,21 (m, 4H), 7,62 (t, 1 H), 7,74 (d, 1 H), 7,81 (d, 1 H), 7,94 (s, 1 H), 8,06 (d, 1H), 8,99 (bs, 1 H), 9,18 (bs, 1H) |
| 11 | 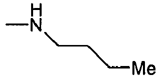 | Et | H | H | 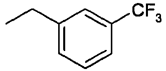 | "0,87 (t, 3H), 1,30 (m, 5H), 1,46 (m, 2H), 2,87 (m, 2H), 3,08 (m, 2H), 3,22 (m, 2H), 3,92 (m, 1 H), 4,11 (m, 1H), 4,24 (m, 2H), 4,51 (m, 2H), 5,85 (bs, 1H), 7,10 (m, 2H), 7,18 (m, 4H), 7,62 (t, 1H), 7,73 (d, 1 H), 7,79 (d, 1 H), 7,92 (s, 1 H), 8,06 (d, 1H), 8,49 (m, 1H), 8,99 (bs, 1H), 9,13 (bs, 1H) |
| 12 | 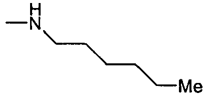 | Et | H | H | 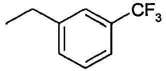 | "0,85 (m, 3H), 1,29 (m, 9H), 1,47 (m, 2H), 2,87 (m, 2H), 3,08 (m, 2H), 3,18 (m, 2H), 3,93 (m, 1H), 4,10 (m, 1 H), 4,24 (m, 2H), 4,51 (m, 2H), 5,84 (d, 1H), 7,11 (m, 2H), 7,19 (m, 4H), 7,66 (t, 1H), 7,73 (d, 1 H), 7,80 (d, 1 H), 7,92 (s, 1 H), 8,07 (d, 1 H), 8,50 (m, 1H), 8,99 (bs, 1H), 9,17 (bs, 1H) |
| 13 | 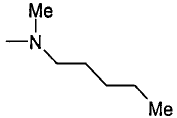 | Et | H | H | 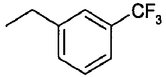 | "0,79 (m, 1H), 0,87 (m, 2H), 1,07 (m, 1H), 1,18 (m, 1 H), 1,26 (m, 1 H), 1,32 (m, 4H), 1,49 (m, 1 H), 1,55 (m, 1 H), 2,86 (m, 2H), 3,08 (m, 2H), 3,28 (m, 1H), 3,28 (m, 1 H), 3,42 (m, 1 H), 3,92 (m, 1H), 4,09 (m, 1H), 4,17 (m, 2H) 4,25 (m, 2H), 5,85 (bs, 1 H), 6,73 (s, 1 H), 7,12 (m, 1 H), 7,20 (m, 4H), 7,61 (t, 1H), 7,73 (d, 1 H), 7,80 (d, 1H), 7,92 (s, 1H), 8,07 (d, 1H), 8,99 (bs, 1H), 9,18 (bs, 1H) |
| 14 | 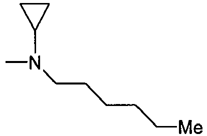 | Et | H | H | 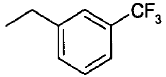 | "0,48 (m, 2H), 0,62 (m, 2H), 0,85 (s, 3H), 1,27 (m, 7H), 1,58 (m, 2H), 2,87 (m, 3H), 3,09 (m, 2H), 3,41 (m, 2H), 3,93 (m, 1H), 4,09 (m, 1H), 4,21 (m, 2H), 4,28 (m, 2H), 5,87 (bs, 1H), 6,87 (m, 1 H), 7,11 (m, 1H), 7,22 (m, 4H), 7,62 (t, 1H), 7,73 (d, 1 H), 7,80 (d, 1H), 7,93 (s, 1 H), 8,03 (d, 1 H), 8,97 (bs, 1H), 9,15 (bs, 1 H) |
| 15 | 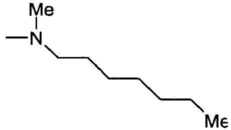 | Et | H | H | 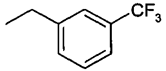 | ^{**}0,84 (m, 3H), 1,22 (m, 11 H), 1,53 (m, 2H), 2,87 (m, 2H), 2,96 (s, 3H), 3,08 (m, 2H), 3,30 (m, 2H), 3,43 (m, 1 H), 3,92 (m, 1 H), 4,20 (m, 4H), 5,83 (bs, 1 H), 6,74 (s, 1H), 7,12 (m, 1 H), 7,21 (m, 4H), 7,61 (t, 1H), 7,73 (d, 1 H), 7,80 (d, 1 H), 7,9 (s, 1 H), 8,08 (d, 1 H), 9,04 (bs, 2H) |
| 16 | 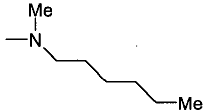 | Et | H | H | 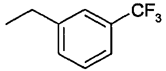 | ^{**}0,84 (m, 3H), 1,23 (m, 9H), 1,53 (m, 2H), 2,92 (m, 2H), 3,08 (s, 3H), 3,12 (m, 2H), 3,31 (m, 1H), 3,44 (m, 1 H), 3,94 (m, 1 H), 4,11 (m, 1 H), 4,18 (m, 2H), 4,27 (m, 2H), 5,88 (bs, 1 H), 6,75 (s, 1 H), 7,13 (m, 1H), 7,23 (m, 4H), 7,63 (dd, 1H), 7,75 (d, 1 H), 7,82 (d, 1 H), 7,95 (s, 1 H), 8,09 (d, 1 H), 8,99 (bs, 1H), 9,19 (bs, 1H) |
| 17 | 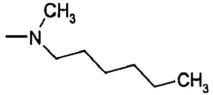 | Et | 3-F | 5-F | 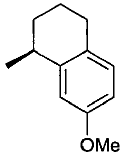 | 0,83 (m, 3H), 1,15 (m, 3H), 1,33 (m, 6H), 1,49 (m, 1H), 1,56 (m,1 H), 1,68 (m, 1H), 1,92 (m, 1H), 2,03 (m, 2H), 2,66 (m, 2H), 2,84 (m, 1 H), 2,96 (m, 4H), 3,14 (m, 2H), 3,30 (m, 1H), 3,44 (m, 1 H), 3,73 (s, 3H), 3,97 (m, 1H), 4,19 (m,3H), 4,52 (m, 1 H), 5,91 (bs, 1 H), 6,79 (s, 1 H), 6,87 (m, 1 H), 6,97 (m 3H), 7,11 (m, 2H), 8,24 (d, 1H), 8,79 (bs, 1H), 8,90 (bs, 1H) |
| 18 | 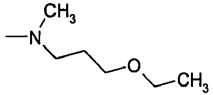 | Et | 3-F | 5-F | 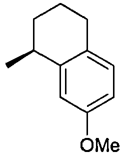 | 1.11 (t, 3H), 1.33 (m, 3H), 1.70 (m, 2H), 1.80 (m, 1 H), 1.91 (m, 1 H), 2.01 (m, 2H), 2.66 (m, 2H), 2.83 (m, 1 H), 2.96 (m, 4H), 3.14 (d, 2H), 3.25 (m, 2H), 3.41 (m, 3H), 3.49 (m, 1 H), 3.72 (s, 3H), 3.94 (m, 1 H), 4.17 (m, 3H), 4.50 (m, 1 H), 5.87 (d, 1H), 6.79 (s, 1H), 6.88 (d, 1H), 6.97 (m, 3H), 7.10 (m, 2H), 8.23 (d, 1H), 8.76 (bs, 1H), 8.85 (bs, 1H) |
| 19 | 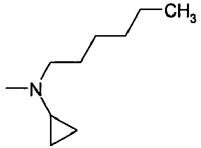 | Et | 3-F | 5-F | 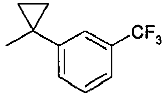 | 0,48 (m, 2H), 0,6 (m, 2H), 0,87 (m, 4H), 1,29 (m, 10H), 1,57 (m, 4H), 2,72 (m, 1H), 2,90 (m, 2H), 3,06 (m, 1 H), 3,44 (m, 3H), 3,90, (m, 1 H), 4,05 (m, 1 H), 4,23 (m, 2H), 5,86 (d, 1 H), 6,95 (m, 4H), 7,57 (dd, 1 H), 7,71 (d, 1H), 7,84 (d, 1H), 7,96 (s, 1H), 8,14 (d, 1H), 9,38 (bs, 1 H), 9,60 (bs, 1 H) |
| 20 | 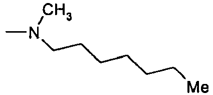 | Et | 3-F | 5-F | 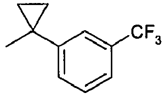 | 0,85 (m, 3H), 1,09 (m, 1 H), 1,19 (m, 4H), 1,32 (m, 8H), 1,46 (m, 4H), 2,72 (m, 1 H), 2,89 (m, 1H), 2,97 (m, 4H), 3,06 (m, 1 H), 3,30 (m, 1 H), 3,45 (m, 1 H), 3,89 (m, 1 H), 4,05 (m, 1 H), 4,18 (m, 2H), 5,86 (bs, 1 H), 6,73 (s, 1 H), 6,94 (m, 3H), 7,56 (m, 1H), 7,70 (d, 1H), 7,83 (d, 1H), 7,95 (s, 1H) 8,16 (d, 1 H), 9,36 (bs, 1 H) 9,59 (bs, 1 H) |
| 21 | 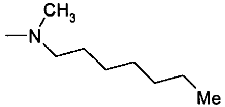 | Et | 3-F | 5-F | 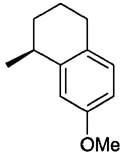 | 0,73-0,80 (2t, 3H), 1,00 (m, 1H), 1,10 (m, 3H), 1,24 (m, 8H), 1,42 (m, 1 H), 1,48 (m, 1 H), 1,62 (m, 1H), 1,84 (m, 1 H), 1,96 (m, 1H), 2,59 (m, 2H), 2,77 (m, 1 H), 2,88 (m, 4H), 3,07 (m, 2H), 3,23 (m, 1 H), 3,37 (t, 1H), 3,65 (s, 3H), 3,88 (m, 1 H) 4,11 (m, 3H), 4,44 (m, 1 H), 5,83 (bs, 1 H), 6,72 (s, 1 H), 6,80 (d, 1 H), 6,90 (m, 3H), 7,02 (d, 1 H), 7,06 (s ; 1H), 8,17 (d, 1H), 8,68 (bs, 1H), 8,80 (bs, 1H) |
| 22 | 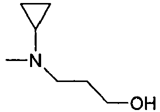 | Et | H | H | 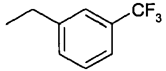 | 0,56 (m, 2H), 0,68 (m, 2H), 1,39 (t, 3H), 1,82 (m, 2H), 2,95 (m, 3H), 3,16 (t, 2H), 3,49 (m, 4H), 3,99 (m, 1 H), 4,16 (m, 1 H), 4,31 (m, 4H), 4,59 (bs, 1 H), 5,97 (bs 1 H), 6,98 (m, 1H), 7,19 (m, 1 H), 7,27 (m, 4H), 7,70 (dd, 1 H), 7,82 (d, 1 H), 7,88 (d, 1H), 8,02 (s, 1H), 8,16 (d, 1H),9,08 (bs, 1H), 9,24 |
| | | | | | | (bs, 1H) |
| 23 | 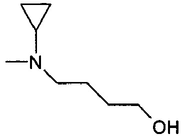 | Et | H | H | 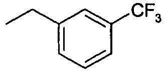 | "0,50 (m, 2H), 0,64 (m, 2H), 1,34 (t, 3H), 1,44 (m, 2H), 1,65 (m, 2H), 2,89 (m, 3H), 3,11 (m, 2H), 3,43 (m, 4H), 3,94 (m, 1 H), 4,12 (m, 1 H), 4,23 (m, 2H), 4,28 (m, 2H), 4,41 (t, 1 H), 5,87 (bs, 1 H), 6,89 (m, 1H), 7,13 (dd, 1H), 7,22 (m, 4H), 7,63 (t, 1H), 7,75 (d,1H), 7,81 (d,1H), 7,95 (s, 1H), 8,05 (d, 1 H), 8,95 (bs, 1H), 9,13 (bs, 1H) |
| 24 | 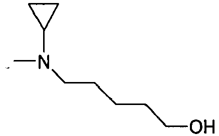 | Et | H | H | 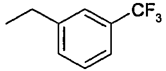 | 0,49 (m, 2H), 0,62 (m, 2H), 1,33 (m, 5H), 1,46 (m, 2H), 1,61 (m, 2H), 2,88 (m, 3H), 3,10 (m, 2H), 3,41 (m, 4H), 3,93 (m, 1 H), 4,11 (m, 1 H), 4,25 (m, 4H), 4,40 (t, 1 H), 5,90 (bs, 1 H), 6,93 (m, 1 H), 7,13 (t, 1H), 7,21 (m, 4H), 7,63 (dd, 1H), 7,76 (d, 1H), 7,81 (d, 1 H), 7,95 (s, 1H), 8,11 (d, 1 H), 8,98 (bs,1H), 9,15 (bs, 1H) |
| 25 | 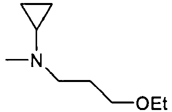 | Et | H | H | 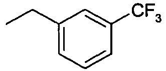 | "0,49 (m, 2H), 0,62 (m, 2H), 1,08 (m, 3H), 1,32 (t, 3H), 1,82 (m, 2H), 2,89 (m, 3H), 3,11 (m, 2H), 3,39 (m, 4H), 3,48 (m, 2H), 3,93 (m, 1 H), 4,09 (m, 1 H), 4,22 (q, 2H), 4,29 (m, 2H), 5,87 (bs 1 H), 6,89 (m, 1H), 7,12 (t, 1H), 7,20 (m, 4H), 7,62 (dd, 1H), 7,74 (d, 1H), 7,80 (d, 1H), 7,94 (s, 1H), |
| | | | | | | 8,03 (d, 1H), 8,97 (bs, 1H), 9,15 (bs, 1H) |
| 26 | 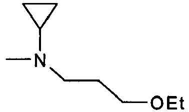 | Et | 3-F | 5-F | 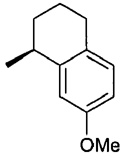 | 0,47 (m, 2H), 0.60 (m, 2H), 1.09 (m, 3H), 1.33 (t, 3H), 1.67 (m, 1 H), 1.87 (m, 3H), 2.02 (m, 2H), 2.67 (m, 2H), 2.89 (m, 3H), 3.13 (d, 2H), 3.40 (m, 6H), 3.95 (m, 1 H), 4.13 (m, 1 H), 4.22 (m, 2H), 4.51 (m, 1 H), 5.91 (d, 1 H), 6.86 (d, 1 H), 6.97 (m, 4H), 7.09 (d, 1H), 7.13 (s, 1H), 8.21 (d, 1H), 8.79 (bs, 1H), 8.90 (bs, 1 H) |
| 27 | 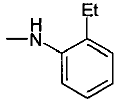 | Et | H | H | 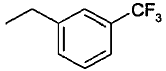 | 1,11 (t, 3H), 1,35 (t, 3H), 2,60 (m, 2H), 2,90 (m, 2H), 3,11 (m, 2H), 3,95 (m, 1H), 4,13 (m, 1H), 4,28 (m, 2H), 4,54 (q, 2H), 5,90 (bs, 1H), 7,13 (m, 1 H), 7,24 (m, 7H), 7,29 (m, 1 H), 7,30 (m, 1 H), 7,64 (dd, 1 H), 7,74 (d, 1H), 7,81 (d, 1 H), 7,94 (s, 1 H), 8,18 (d, 1 H), 8,99 (bs, 1 H), 9,18 (bs, 1 H), 9,98 (s, 1H) |
| 28 | 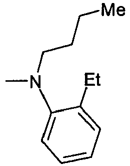 | Et | H | H | 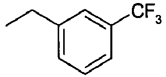 | 0,88 (t, 3H), 1,03 (m, 3H), 1,31 (m, 2H), 1,40 (m, 3H), 1,52 (m, 2H), 2,36 (m, 1 H), 2,51 (m, 1 H), 2,79 (m, 2H), 3,01 (m, 2H), 3,36 (m, 1 H), 3,86 (m, 1 H), 3,96 (m, 2H), 4,22 (m, 2H), 4,39 (m, 2H), 5,81 (bs, 1H), 7,10-77,25 (m, 8H), 7,33 (m, 2H), 7,60 (m, 1 H), 7,76 (m, 2H), 7,92 (m, 2H), 8,92 (bs, 1 H), 9,08 (bs, 1 H) |
| 29 | 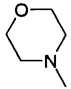 | Et | H | H | 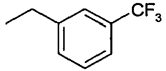 | 1,35 (t, 3H), 2,89 (m, 2H), 3,10 (m, 2H), 3,46-3,63 (m, 8H), 3,92 (m, 1 H), 4,11 (m, 1 H), 4,29 (m, 4H), 5,87 (bs, 1H), 6,73 (s, 1 H), 7,13 (m, 1 H), 7,21 (m, 4H), 7,62 (m, 1 H), 7,74 (d, 1 H), 7,81 (d, 1 H), 7,94 (s, 1 H), 8,12 (d, 1 H), 9,08 (bs, 2H) |
| 30 | 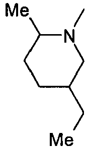 | Et | H | H | 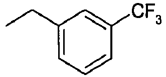 | **0,82 (m, 3H), 1,15-1,87 (m, 16H), 2,86 (m, 2H), 3,09 (m, 2H), 3,92 (d, 1 H), 4,12 (m, 3H), 4,27 (m, 2H), 5,88 (s, 1 H), 6,62 (s, 1 H), 7,17 (m, 5H), 7,61 (m, 1 H), 7,72 (d, 1 H), 7,80 (d, 1 H), 7,93 (s, 1 H), 8,07 (m, 1 H), 8,99 (bs, 1H), 9,20 (bs, 1 H) |
| 31 | 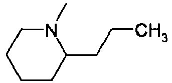 | -(CH₂)₃-CH₃ | H | H | 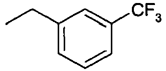 | 0,81-1,77 (m, 20H), 2,89 (m, 2H), 3,10 (m, 3H), 3,50 (m, 1 H), 3,94 (m, 1 H), 4,13 (m, 3H), 4,27 (m, 2H), 4,50-4,73 (m, 1 H), 5,88 (bs, 1 H), 6,64 (s, 1 H), 7,14 (m, 1 H), 7,24 (m, 4H), 7,64 (m, 1 H), 7,75 (d, 1H), 7,82 (d, 1H), 7,95 (s, 1H), 8,12 (m, 1 H), 9,08 (bs, 2H) |
| 32 | 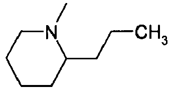 | 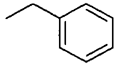 | H | H | 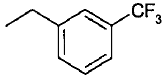 | 0,72-1,70 (m, 13H), 2,74-2,98 (m, 3H), 3,14 (m, 2H), 3,34 (m, 1 H), 3,92 (m, 1H), 4,12 (m, 1H), 4,30 (m, 2H), 4,64 (m, 1 H), 5,46 (s, 2H), 5,90 (bs, 1H), 6,72 (s, 1H), 7,19 (m, 7H), 7,36 (m, 3H), |
| | | | | | | 7,65 (m, 1H), 7,77 (d, 1H), 7,83 (d, 1H), 7,97 (s, 1H), 8,21 (d, 1H), 8,96 (bs, 1H), 9,14 (bs, 1 H) |
| 33 | 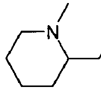 | Et | H | H | 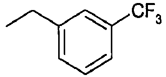 | 0,77-1,80 (m, 16H), 2,84 (m, 2H), 3,10 (m, 3H), 3,50 (m, 1 H), 3,92 (m, 1H), 4,12 (m, 3H), 4,28 (m, 2H), 4,71 (m, 1 H), 5,90 (d, 1H), 6,62 (s, 1 H), 7,22 (m, 5H), 7,62 (m, 1 H), 7,74 (d, 1 H), 7,81 (d, 1 H), 7,94 (s, 1H), 8,11 (d, 1H), 8,99 (bs, 1H), 9,19 (bs, 1 H) |
| 34* | 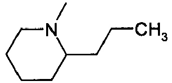 | Et | H | H | 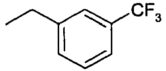 | 0,70-1,85 (m, 16H), 2,87 (m, 2H), 3,11 (m, 3H), 3,48 (m, 1 H), 3,94 (m, 1H), 4,15 (m, 3H), 4,27 (m, |
| | Isomère A | | | | | 2H), 4,72 (m, 1 H), 5,90 (d, 1 H), 6,64 (s, 1 H), 7,14 (m, 1H), 7,22 (m, 4H), 7,63 (m, 1H), 7,74 (d, 1H), 7,83 (d, 1H), 7,96 (s, 1H), 8,12 (d, 1H), 9,09 (bs, 2H) |
| 35* | 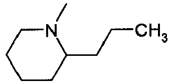 Isomère B | Et | H | H | 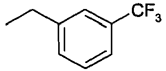 | 0,70-1,85 (m, 16H), 2,90 (m, 2H), 3,13 (m, 3H), 3,50 (m, 1 H), 3,96 (m, 1 H), 4,14 (m, 3H), 4,28 (m, 2H), 4,72 (m, 1 H), 5,94 (bs, 1 H), 6,63 (s, 1 H), 7,14 (m, 1 H), 7,23 (m, 4H), 7,63 (m, 1H), 7,75 (d, 1H), 7,84 (d, 1 H), 7,97 (s, 1 H), 8,11 (d, 1 H), 9,04 (bs, 1H), 9,30 (bs, 1 H) |
| 36 | 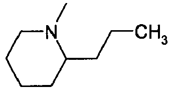 | Et | H | H | 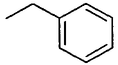 | 0,7-1,87 (m, 16H), 2,89 (m, 2H), 3,05 (d, 1 H), 3,15 (m, 2H), 3,52 (m, 1H), 3,95 (m, 1H) ; 4,17 (m, 5H), 4,75 (m, 1 H), 5,88 (bs, 1 H), 6,68 (s, 1 H), 7,18 (m, 1H), 7,25 (m, 4H), 7,40 (m, 3H), 7,53 (m, 2H), 8,13 (m, 1H), 9,00 (bs, 2H) |
| 37 | 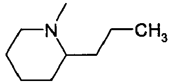 | Et | H | H | 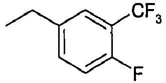 | 0,7-1,85 (m, 16H), 2,88 (m, 2H), 3,11 (m, 3H), 3,51 (m, 1H), 3,92 (m, 1H), 4,19 (m, 5H), 4,74 (m, 1H), 5,90 (bs, 1 H), 6,65 (s, 1 H), 7,16 (m, 1H), 7,23 (m, 4H), 7,58 (m, 1H), 7,90 (m, 1H), 8,03 (d, 1H), 8,13 (m, 1H), 8,96 (bs, 1H), 9,16 (bs, 1 H) |
| 38 | 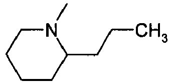 | Et | H | H | 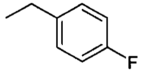 | 0,79-1,88 (m, 16H), 2,86 (m, 2H), 3,03 (m, 1H), 3,08 (m, 1 H), 3,14 (m, 1H), 3,52 (m, 1 H), 3,93 (m, 1H), 4,16 (m, 5H), 4,73 (m, 1 H), 5,87 (bs, 1 H), 6,66 (s, 1H), 7,15 (m, 1H), 7,27 (m, 6H), 7,57 (m, 2H), 8,12 (d, 1H), 8,94 (bs, 2H) |
| 39 | 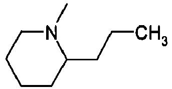 | Et | H | H | 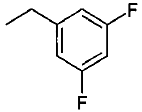 | 0,77-1,90 (m, 16H), 2,85 (m, 2H), 3,02 (m, 1H), 3,11 (m, 2H), 3,48 (m, 1 H), 3,91 (m, 1 H), 4,09 (m, 1 H), 4,17 (m, 4H), 4,71 (m, 1 H), 5,88 (bs, 1 H), 6,61 s, 1H), 7,12 (m, 1H), 7,24 (m, 7H), 8,10 (d, 1H), 8,98 (bs, 1H), 9,26 (bs, 1H) |
| 40 | 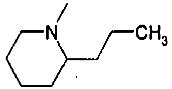 | Et | H | H | 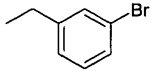 | 0,77-1,77 (m, 16H), 2,85 (m, 2H), 3,04 (m, 1H), 3,10 (m, 2H), 3,48 (m, 1 H), 3,92 (m, 1H), 4,09 (m, 1 H), 4,17 (m, 4H), 4,71 (m, 1 H), 5,88 (bs, 1 H), 6,63 (s, 1H), 7,12 (m,1H), 7,20 (m, 4H), 7,33 (m, 1 H), 7,51 (d, 1 H), 7,57 (d, 1H), 7,77 (s, 1 H), 8,10 (d, 1H), 8,95 (bs, 1H), 9,18 (bs, 1H) |
| 41 | 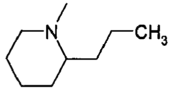 | Et | H | H | 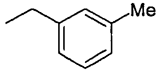 | 0,86-1,62 (m, 16H), 2,27 (s, 3H), 2,89 (m, 2H), 3,01 (m, 1 H), 3,11 (m, 2H), 3,49 (m, 1 H), 3,92 (m, 1 H), 4,13 (m, 5H), 4,71 (m, 1 H), 5,87 (bs, 1 H), 6,63 (s, 1H), 7,21 (m, 9H), 8,10 (bs, 1H), 8,87 (bs, 1H), 9,03 (bs, 1H) |
| 42 | 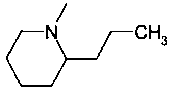 | Et | H | H | 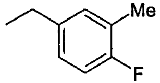 | 0,86-1,58 (m, 16H), 2,19 (s, 3H), 2,85 (m, 2H), 2,99 (m, 1 H), 3,11 (m, 2H), 3,47 (m, 1 H), 3,89 (m, 1 H), 4,13 (m, 5H), 4,71 (m, 1 H), 5,86 bs, 1H), 6,63 (s, 1H), 7,12 (m, 2H), 7,25 (m, 4H), 7,35 (m, 1H), 7,41 (m, 1H), 8,09 (d, 1H), 8,97 (bs, 2H) |
| 43 | 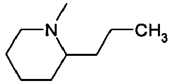 | Et | H | H | 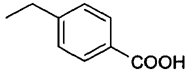 | 0,77-1,77 (m, 16H), 2,76(m, 1H), 2,86 (m, 1 H), 2,92 (m, 1 H), 3,03 (m, 2H), 3,47 (m, 1 H), 3,84 (m, 1 H), 4,14 (m, 5H), 4,70 (m, 1 H), 5,69 (bs, 1 H), 6,63 (s, 1H), 7,12 (m, 1H), 7,19 (m, 4H), 7,58 (m, 2H), 7,91 (m, 2H), 8,09 (bs, 1H), 9,20 (bs, 1H) |
| 44 | 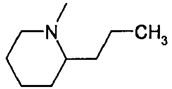 | Et | H | H | 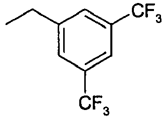 | 0,78-1,77 (m, 16H), 2,83 (m, 2H), 3,13 (m, 3H), 3,49 (m, 1 H), 3,89 (m, 1 H), 4,11 (m, 3H), 4,45 (m, 2H), 4,71 (m, 1 H), 5,93 (bs, 1 H), 6,61 (m, 1 H), 7,12 (m, 1H), 7,21 (m, 4H), 8,13 (m, 2H), 8,30 (s, 2H), 9,11 (bs, 1H), 9,40 (bs, 1H) |
| 45 | 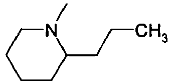 | Et | H | H | 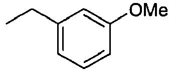 | 0,75-1,85 (m, 16H), 2,2,85 (m, 3H), 3,03 (m, 1H), 3,12 (m, 1 H), 3,49 (m, 1 H), 3,75 (s, 3H), 3,95 (m, 1 H), 4,13 (m, 5H), 4,72 (m, 1H), 5,92 (d, 1 H), 6,63 (m, 1H), 6,92 (d, 1H), 7,06 (d, 1H), 7,20 (m, 6H), 7,56 (t, 1H), 8,11 (bs, 1 H), 8,96 (bs, 1H), 9,24 (bs, 1H) |
| 46 (HCl)₂ | 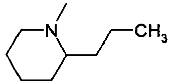 | Et | H | H | 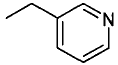 | 0,7-1,85 (m, 16H), 2,91 (m, 2H), 3,13 (m, 3H), 3,70 (m, 2H), 3,97 (m, 1H), 4,15 (m, 4H), 4,32 (m, 2H), 4,74 (m, 1 H), 6,66 (s, 1 H), 7,14 (m, 1 H), 7,22 (m, 4H), 7,71 (m, 1H), 8,14 (m, 1H), 8,29 (m, 1 H), 8,73 (d, 1 H), 8,87 (s, 1 H), 9,14 (bs, 1 H), 9,50 (bs, 1 H) |
| 47 | 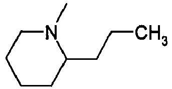 | Et | 3-F | 5-F | 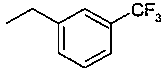 | 0,76-1,85 (m, 16H), 2,88 (m, 2H), 3,11 (m, 3H), 3,49 (m, 1 H), 3,91 (m, 1 H), 4,14 (m, 3H), 4,30 (m, 2H), 4,72 (m, 1 H), 5,91 (bs, 1 H), 6,65 (s, 1 H), 6,98 (m, 3H), 7,64 (m, 1H), 7,76 (d, 1H), 7,82 (d, |
| | | | | | | 1 H), 7,95 (s, 1 H), 8,23 (d, 1 H), 9,01 (bs, 1 H), 9,21 (bs, 1H) |
| 48 | 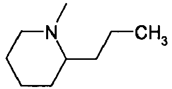 | Et | H | H | 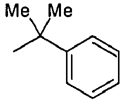 | 0,7-1,85 (m, 22H), 2,5 (m, 1H), 2,65 (m, 1H), 2,82 (m, 1 H), 3,00 (m, 1 H), 3,15 (m, 1 H), 3,47 (m, 1H), 3,82 (m, 1 H), 4,02 (m, 1 H), 4,16 (m, 2H), 4,71 (m, 1 H), 5,80 (d, 1H), 6,59 (s, 1 H), 7,15 (m, 5H), 7,32 (m, 3H), 7,56 (d, 2H), 8,04 (m, 1 H), 8,94 (bs, 1H), 9,24 (bs, 1H) |
| 49 | 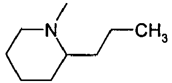 | Et | H | H | 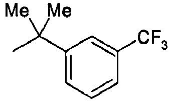 | 0,7-1,85 (m, 22H), 2,5 (m, 1 H), 2,66 (m, 1 H), 2,82 (m, 1H), 3,00 (m, 1 H), 3,15 (m, 1 H), 3,46 (m, 1 H), 3,86 (m, 1H), 4,04 (m, 1 H), 4,15 (m, 2H), 4,71 (m, 1 H), 5,81 (d, 1 H), 6,57 (s, 1 H), 7,06 (m, 1 H), 7,19 (m, 4H), 7,61 (m, 1H), 7,70 (m, 1H), 7,92 (m, 2H), 8,02 (d, 1H), 9,14 (bs, 1H), 9,55 (bs, 1H) |
| 50 | 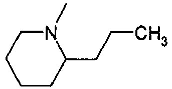 | Et | 3-F | 5-F | 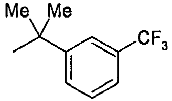 | 0,7-1,85 (m, 22H), 2,5 (m, 1 H), 2,70 (m, 1 H), 2,86 (m, 1 H), 3,03 (d, 1 H), 3,15 (m, 1 H), 3,49 (m, 1H), 3,85 (m, 1 H), 4,05 (m, 1 H), 4,16 (m, 2H), 4,72 (m, 1H), 5,82 (d, 1 H), 6,58 (s, 1H), 6,91 (d, 1H), 6,97 (m, 2H), 7,64 (m, 1 H), 7,70 (d, 1 H), 7,91 (d, 1H), 7,95 (s, 1H), 8,12 (d, 1H), 9,11 (bs, 1H), 9,47 (bs, 1H) |
| 51 | 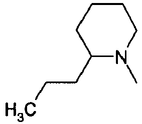 | Et | H | H | 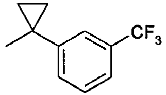 | 0,70-1,85 (m, 20H), 2,72 (m, 1 H), 2,80 (m, 1H), 2,91 (m, 1 H), 3,05 (d, 1 H), 3,15 (m, 1 H), 3,49 (m, 1H), 3,91 (m, 1 H), 4,02 (m, 1 H), 4,15 (m, 2H), 4,71 (m, 1 H), 5,83 (bs, 1 H), 6,57 (s, 1 H), 7,11 (m, 1 H), 7,20 (m, 4H), 7,54 (m, 1H), 7,67 (m, 1 H), 7,82 (m, 1 H), 7,93 (s, 1 H), 8,05 (d, 1 H), 9,40 (bs, 1H), 9,70 (bs, 1 H) |
| 52 | 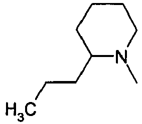 | Et | prop-2-èn-1-yloxy | H | 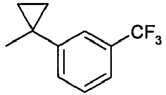 | 0,70-1,85 (m, 20H), 2,58 (m, 1 H), 2,66 (m, 1 H), 2,77 (m, 1 H), 2,85 (m, 1 H), 3,00 (m, 1H), 3,33 (m, 1H), 3,71 (m, 1H), 3,85 (m, 1H), 4,00 (m, 2H), 4,26 (m, 2H), 4,55 (m, 1 H), 5,03 (d, 1 H), 5,14 (dd, 1H), 5,66 (bs, 1 H), 5,80 (m, 1 H), 6,42 (s, 1 H), 6,54 (d, 1 H), 6,59 (d, 1 H), 6,63 (s, 1 H), 6,94 (m, 1 H), 7,40 (m, 1 H), 7,52 (d,1 H), 7,66 (d, 1 H), 7,77 (s, 1H), 7,90 (m, 1H), 9,16 (bs, 1H), 9,16 (bs, 1H) |
| 53 | 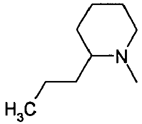 | Et | 3-F | 5-F | 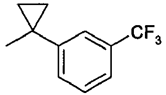 | 0,70-1,85 (m, 20H), 2,70 (m, 1H), 2,86 (m, 1H), 2,94 (m, 1 H), 3,05 (d, 1 H), 3,14 (m, 1H), 3,47 (m, 1H), 3,88 (m, 1 H), 4,03 (m, 1 H), 4,14 (m, 2H), 4,70 (m, 1 H), 5,82 (bs, 1 H), 6,58 (s, 1H), 6,92 (m, 3H), 7,55 (m, 1 H), 7,68 (m, 1H), 7,81 (d, 1 H), 7,93 (s, 1H), 8,09 (m, 1H), 9,37 (bs, 1H), 9,62 |
| | | | | | | (bs, 1 H) |
| 54 | 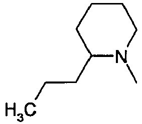 | Et | H | H | 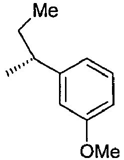 | 0,70-1,85 (m, 19H), 1,91 (m, 1H), 2,10 (m, 1H), 2,85 (m, 3H), 3,05 (d, 1 H), 3,15 (m, 1 H), 3,49 (m, 1 H), 3,75 (s, 3H), 3,89 (m, 1 H), 3,98 (m, 1 H), 4,14 (m, 3H), 4,71 (m, 1 H), 5,75 (d, 1 H), 6,60 (s, 1H), 6,91 (d, 1H), 7,01 (d, 1H), 7,11 (m, 2H), 7,17 (m, 4H), 7,28 (m, 1 H), 8,01 (d, 1 H), 8,81 (bs, 1 H), 9,20 (bs, 1H) |
| 55 | 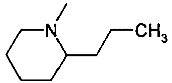 | Et | H | H | 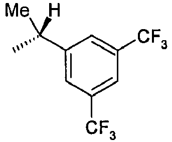 | 0,70-1,80 (m, 19H), 2,80 (m, 2H), 2,98 (m, 2H), 3,07 (m, 1 H), 3,41 (m, 1 H), 3,87 (m, 1H), 3,95 (m, 1H), 4,11 (m, 2H), 4,63 (m, 2H), 5,74 (d, 1H), 6,52 (s, 1H), 7,03 (m, 1H), 7,10 (m, 4H), 8,00 (d, 1 H), 8,11 (s, 1 H), 8,23 (s, 2H), 8,94 (bs, 1H), 9,54 (bs, 1H) |
| 56 | 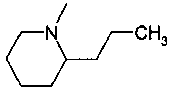 | Et | H | H | 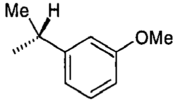 | 0,60-1,85 (m, 19H), 2,79 (m, 3H), 2,99 (m, d, 1 H), 3,08 (m, 1 H), 3,41 (m, 1 H), 3,68 (s, 3H), 3,82 (m, 1 H), 3,94 (m, 1 H), 4,09 (m, 2H), 4,29 (m, 1 H), 4,64 (m, 1 H), 5,69 (bs, 1 H), 6,54 (s, 1 H), 6,83 (d, 1H), 6,97 (d, 1H), 7,04 (m, 2H), 7,10 (m, 4H), 7,20 (m, 1 H), 7,96 (m, 1H), 8,71 (bs, 1H), 9,21 (bs, 1 H) |
| 57 | 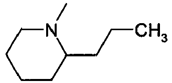 | Et | H | H | 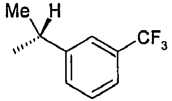 | 0,55-1,70 (m, 19H), 2,71 (m, 2H), 2,80 (m, 1H), 2,91 (d, 1 H), 3,00 (m, 1 H), 3,34 (m,1 H), 3,77 (m, 1 H), 3,87 (m, 1H), 4,01 (m, 2H), 4,42 (m, 1H), 4,56 (m, 1 H), 5,65 (d, 1 H), 6,45 (s, 1 H), 6,96 (m, 1H), 7,02 (m, 4H), 7,48 (m, 1 H), 7,59 (d, 1 H), 7,69 (d, 1 H), 7,80 (s, 1 H), 7,90 (d, 1H), 8,80 (bs, 1 H), 9,35 (bs, 1 H) |
| 58 | 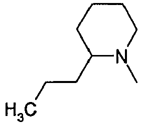 | Et | H | H | 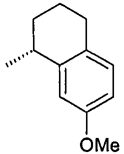 | 0,7-1,85 (m, 17H), 1,90 (m, 1H), 2,02 (m, 2H), 2,63 (m, 1 H), 2,68 (m, 1H), 2,90 (m, 2H), 3,10 (m, 3H), 3,47 (m, 1 H), 3,71 (s, 3H), 3,95 (m, 1 H), 4,14 (m, 3H), 4,52 (m, 1H), 4,71 (m, 1H), 5,92 (d, 1 H), 6,00 (s, 1H), 6,83 (d, 1 H), 7,06 (d, 1 H), 7,16 (m, 6H), 8,15 (d, 1H), 8,69 (bs, 1 H), 9,03 (bs, 1H) |
| 59 | 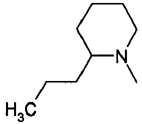 | Et | H | H | 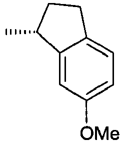 | 0,70-1,85 (m, 16H), 2,15 (m, 1H), 2,45 (m, 1H), 2,77 (m, 1H), 2,89 (m, 2H), 2,98 (m, 2H), 3,11 (m, 2H), (m, 1 H), 3,71 (s, 3H), 3,91 (m, 1 H), 4,15 (m, 3H), 4,71 (m, 1H), 4,77 (m, 1 H), 5,88 (bs, 1 H), 6,60 (s, 1H), 6,87 (d, 1H), 7,18 (m, 7H), 8,12 (m, 1H), 8,91 (bs, 1H), 9,17 (bs, 1H) |
| 60 | 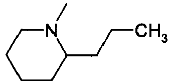 | Et | H | H | 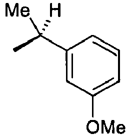 | 0,70-1,85 (m, 19H), 2,77 (m, 2H), 2,84 (m, 1H), 3,06 (d, 1 H), 3,14 (m, 1 H), 3,47 (m, 1 H), 3,71 (s, 3H), 3,83 (m, 1 H), 4,06 (m, 1 H), 4,13 (m, 2H), 4,32 (m, 1H), 4,70 (m, 1 H), 5,84 (bs, 1 H), 6,56 (s, 1H), 6,84 (d, 1H), 7,02 (d, 1 H), 7,11 (m, 2H), 7,17 (m, 4H), 7,22 (m, 1H), 8,02 (d, 1H), 9,03 (bs, 2H) |
| 61 | 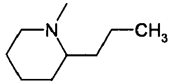 | Et | H | H | 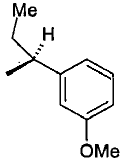 | ** 0,65 (t, 3H), 0,70-1,90 (m, 17H), 2,15 (m, 1H), 2,81 (m, 2H), 2,83 (m, 1 H), 3,05 (d, 1H), 3,14 (m, 1 H), 3,48 (m, 1 H), 3,71 (s, 3H), 3,81 (m, 1H), 4,05 (m, 1H), 4,13 (m, 3H), 4,70 (m, 1H), 5,82 (d, |
| | | | | | | 1 H), 6,56 (s, 1 H), 6,85 (d, 1 H), 6,99 (d, 1 H), 7,11 (m, 2H), 7,18 (m, 4H), 7,23 (m, 1 H), 8,02 (m, 1 H), 9,03 (bs, 2H) |
| 62 | 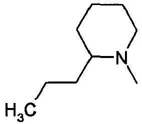 | Et | H | H | 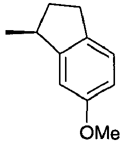 | **0,70-1,80 (m, 18H), 2,16 (m, 1H), 2,43 (m, 1 H), 2,75 (m, 1 H), 2,82 (m, 2H), 2,96 (m, 1 H), 3,09 (m, 3H), 3,47 (m, 1 H), 3,90 (m, 1 H), 4,14 (m, 3H), 4,73 (m, 2H), 5,84 (bs, 1H), 6,63 (s, 1 H), 6,88 (d, 1H), 7,12 (m, 1H), 7,21 (m, 6H), 8,10 (m, 1H), 8,89 (bs, 1H), 9,04 (bs, 1H) |
| 63 | 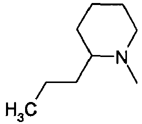 | Et | H | H | 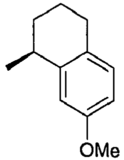 | **0,70-1,80 (m, 17H), 1,89 (m, 1H), 2,00 (m, 2H), 2,64 (m, 2H), 2,88 (m, 2H), 3,11 (m, 3H), 3,48 (m, 1 H), 3,71 (s, 3H), 3,95 (m, 1 H), 4,14 (m, 3H), 4,49 (m, 1 H), 4,70 (m, 1 H), 5,87 (bs, 1 H), 6,62 (s, 1H), 6,85 (d, 1H), 7,07 (d, 1H), 7,12 (m, 2H), 7,23 (m, 4H), 8,08 (d, 1H), 8,74 (bs, 1H), 8,82 (bs, 1H) |
| 64 | 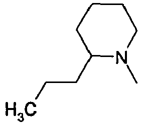 | Et | 3-F | 5-F | 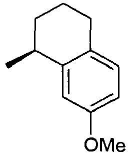 | 0,60-2,05 (m, 20H), 2,60 (m, 2H), 2,77 (m, 1 H), 2,85 (m, 1 H), 3,07 (m, 3H), 3,48 (m, 1 H), 3,65 (s, 3H), 3,88 (m, 1 H), 4,09 (m, 3H), 4,44 (m, 1 H), 4,64 (m, 1 H), 5,83 (bs, 1 H), 6,59 (s, 1 H), 6,79 (d, 1 H), 6,92 (m, 3H), 7,03 (m, 2H), 8,17 (m, 1 H), 8,70 (bs, 1H), 8,80 (bs, 1H) |
| 65 | 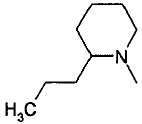 | Et | 3-F | 5-F | 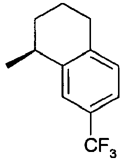 | 0.70-2.20 (m, 20H), 2.86 (m, 4H), 3.14 (m, 3H), 3.50 (m, 1 H), 3.98 (m, 1 H), 4.15 (m, 3H), 4.68 (m, 2H), 5.92 (d, 1 H), 6.67 (s, 1 H), 6.99 (m, 3H), 7.42 (d, 1 H), 7.64 (d, 1 H), 7.95 (s, 1H), 8.24 (d, 1 H), 8.85 (bs, 1 H), 9.00 (bs, 1H) |
| 66 | 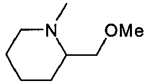 | Et | H | H | 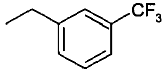 | 1,36 (m, 4H), 1,66 (m, 5H), 2,88 (m, 2H), 3,10 (m, 2H), 3,21 (m, 4H), 3,48 (m, 1 H), 3,73 (m, 1 H), 3,92 (m, 1H), 4,12 (m, 4H), 4,27 (m, 3H), 5,90 (bs, 1H), 6,65 (s, 1 H), 7,14 (m, 1 H), 7,22 (m, 4H), 7,63 (m, 1H), 7,75 (d, 1H), 7,82 (d, 1H), 7,95 (s, |
| | | | | | | 1H), 8,14 (d, 1 H), 8,19 (bs, 1H), 9,00 (bs, 1H) |
| 67 | 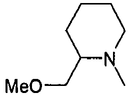 | Et | 3-F | 5-F | 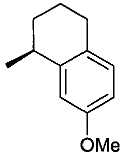 | **1,36 (m, 4H), 1,63 (m, 6H), 1,90 (m, 1H), 2,02 (m, 1 H), 2,65 (m, 2H), 2,84 (m, 2H), 2,93 (m, 1 H), 3,20 (m, 5H), 3,25 (s, 3H), 3,19 (m, 4H), 3,29 (s, 3H), 3,94 (m, 1 H), 4,14 (m, 3H), 4,29 (m, 1 H), 4,50 (m, 1 H), 5,86 (d, 1 H), 6,65 (s, 1 H), 6,86 (d, 1H), 6,97 (m, 3H), 7,08 (d, 1H), 7,12 (s, 1H), 8,16 (d, 1H), 8,74 (bs, 1H), 8,83 (bs, 1H) |

| | | | | | | |
|---|---|---|---|---|---|---|
| *La stéréochimie exacte au niveau de l'amide coniine des composés 34 et 35 n'a pas été déterminée. | | | | | | |

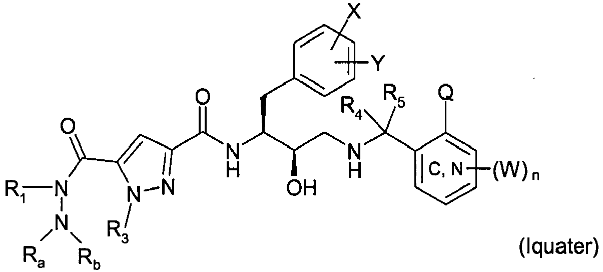

**Tableau II**

| | R₁ | -NRₐR_{b} | R₃ | X | Y | 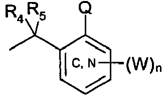 | |
|---|---|---|---|---|---|---|---|
| 68 | H | 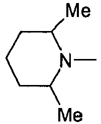 | Et | H | H | 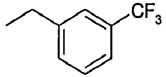 | 0,97 (m, 6H), 1,32 (m, 6H), 1,66 (m, 3H), 2,51 (m, 1H), 2,61 (m, 1H), 2,89 (m, 2H), 3,11 (m, 2H), 3,95 (m, 1 H), 4,13 (m, 1 H), 4,28 (m, 2H), 4,51 (m, 2H), 5,94 (bs, 1H), 7,20 (m, 6H), 7,64 (t, 1 H), 7,75 (d, 1 H), 7,82 (d, 1H), 7,95 (s, 1 H), 8,15 (d, 1 H), 8,62 (bs, 1 H), 9,09 (bs, 1 H), 9,70 (bs, 1H) |
| 69 | H | 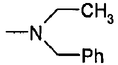 | Et | H | H | 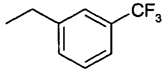 | 1,05 (t, 3H), 1,21 (t, 3H), 2,90 (m, 4H), 3,10 (m, 1H), 3,13 (m, 1 H), 3,93 (m, 1 H), 4,00 (m, 2H), 4,09 (m, 1 H), 4,26 (m, 2H), 4,38 (m, 2H), 5,96 (bs, 1H), 6,95 (s, 1H), 7,12 (m, 1H), 7,24 |
| | | | | | | | (m, 5H), 7,29 (dd, 2H), 7,38 (d, 2H), 7,62 (m, 1 H), 7,73 (d, 1 H), 7,81 (d, 1 H), 7,95 (s, 1 H), 8,14 (d, 1 H), 9,01 (bs, 1 H), 9,25 (bs, 1H), 9,42 (s, 1H) |
| 70 | H | 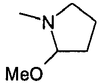 | Et | H | H | 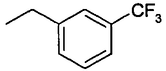 | 1,27 (t, 3H), (m, 1 H), (m, 2H), (m, 1 H), 2,82 (m, 3H), 3,02 (m, 3H), 3,14 (m, 5H), 3,31 (m, 1H), 3,88 (m, 1 H), 4,04 (m, 1 H), 4,20 (m, 2H), 4,43 (m, 2H), 5,85 (bs, 1 H), 7,02 (s, 1 H), 7,06 (m, 1H), 7,13 (m, 4H), 7,57 (m, 1H), 7,68 (d, 1 H), 7,75 (d, 1 H), 7,88 (s, 1 H), 8,09 (d, 1 H), 8,93 (bs, 1 H), 9,18 (bs, 1H), 9,55 (s, 1 H) |
| 71 | H | 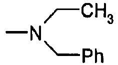 | Et | 3-F | 5-F | 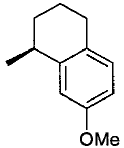 | 1,05 (t, 3H), 1,21 (t, 3H), 1,67 (m, 1H), 1,92 (m, 1 H), 2,02 (m, 2H), 2,65 (m, 2H), 2,81 (m, 1H), 2,92 (m, 3H), 3,13 (m, 2H), 3,69 (s, 3H), 3,98 (m, 3H), 4,14 (m, 1H), 4,38 (m, 2H), 4,51 (m, 1 H), 5,90 (bs, 1 H), 6,87 (m, 1 H), 6,96 (m, 4H), 7,09 (d, 1H), 7,15 (d, 1H), 7,23 (m, 1H), 7,28 (m, 2H), 7,38 (m, 2H), 8,23 (d, 1 H), 8,85 (bs, 1H), 9,01 (bs, 1H), 9,44 (bs, 1H) |
| 72 | H | 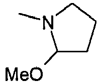 | Et | 3-F | 5-F | 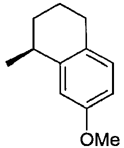 | 1,27 (t, 3H), 1,40 (m, 1 H), 1,65 (m, 3H), 1,85 (m, 2H), 1,95 (m, 2H), 2,58 (m, 2H), 2,84 (m, 3H), 3,09 (m, 7H), 3,30 (m, 1 H), 3,64 (m, 4H), 3,90 (m, 1 H), 4,07 (m, 1 H), 4,43 (m, 3H), 5,85 (bs, 1 H), 6,81 (m, 1 H), 6,90 (m, 3H), 7,02 (m, 2H), 7,08 (s, 1 H), 8,19 (d, 1 H), 8,74 (bs, 1 H), 8,89 (bs, 1 H), 9,54 (s, 1 H) |

Les composés de l'invention ont fait l'objet d'essais pharmacologiques qui ont montré leur intérêt comme substances actives en thérapeutique.

Ils ont en particulier été testés quant à leurs effets d'inhibition de la production du peptide β-amyloïde (β-A4).

Le peptide β-amyloïde (β-A4) est un fragment d'une protéine précurseur plus importante appelée APP (Amyloid Precursor Protein), Cette dernière est produite et présente dans différentes cellules de tissu animal et humain. Toutefois, son clivage, dans le tissu cérébral, par des enzymes de type protéase, conduit à la formation du peptide β-A4 qui s'accumule sous forme de plaque amyloïde. Les deux protéases responsables de la production du peptide amyloïde sont connues sous le nom de beta et gamma-secrétases (M.S. Wolfe, J. Med. Chem., 2001, 44, 2039-60).

Or il a été démontré que ce dépôt progressif du peptide β-A4 est neurotoxique et pourrait jouer un rôle important dans la maladie d'Alzheimer (C. Hölscher, Neurosciences, 2005, 16, 181-212).

Ainsi, les composés de la présente invention, en tant qu'inhibiteur de la production du peptide β-amyloïde (β-A4) par inhibition de la beta secrétase BACE1 (β-site APP cleavage enzyme subtype 1), peuvent être utilisés dans le traitement de pathologies comme la démence sénile, la maladie d'Alzheimer, le syndrome de Down, la maladie de Parkinson, l'angiopathie amyloïde, les désordres cérébro-vasculaires, et/ou les maladies neurodégénératives (F. Coria et al, Neuropath. Appl. Neurobiol., 1996, 22, 216-227).

L'activité enzymatique de BACE1 et la production du peptide β-A4 peuvent être analysés *in vitro* et *in vivo.*

Différents essais peuvent être utilisés pour démontrer l'activité inhibitrice de composés sur BACE1. Selon le cas, ces essais peuvent être réalisés avec BACE1 soluble et un substrat synthétique, avec des cellules exprimant le substrat APP et BACE1 et/ou avec des modèles animaux sauvages ou transgéniques exprimant APP et BACE1. Les cellules peuvent être de différents types, soit transfectées avec le gène de l'APP sauvage ou muté (CHO, HEK293, SHSY5Y, H4,...), soit naturelles (IMR32, cultures primaires de neurones de souris,...). De la même façon, les tests de sélectivité vis-à-vis de la Cathepsine D et de l'alpha sécrétase notamment peuvent être réalisés (I. Hussain et al, Journal of Neurochemistry, 2007, 100, 802-809).

De tels essais sont décrits, selon les protocoles ci-après.

Dans ce qui suit :
- DMSO = diméthylsulfoxyde
- DMEM/F12 = Dulbecco's Modified Eagle's Medium / mélange nutritif F12 de Ham

### Test 1 Inhibition de BACE1 soluble

Ce test utilise BACE1 soluble et un substrat synthétique de BACE1.

L'enzyme soluble BACE1 humaine recombinante est disponible chez R&D Systems (référence 931-AS).

Le peptide synthétique, constitué par l'enchaînement d'acides aminés Ser-Glu-Val-Asn-Leu-Asp-Ala-Glu-Phe-Arg (S-10-R, disponible chez NeoMPS, référence SP000122), est utilisé comme substrat synthétique de BACE1.

Ce substrat synthétique incorpore le site de coupure de BACE1 et des groupements fluorescents ou chromogéniques, afin que les produits de coupure puissent ensuite être détectés, soit par UV après séparation HPLC, soit par mesure de fluorescence ou de densité optique. Dans le cas présent, le produit de coupure Asp-Ala-Glu-Phe-Arg, libéré lors de l'hydrolyse de S-10-R par BACE1, est quantifié par détection UV [195nm] après séparation par HPLC avec un gradient d'acétonitrile de 2% à 100% [H1100, Agilent ; Colonne Atlantis dC18 (5mmx2,1mm ; 3µm)].

Le substrat synthétique S-10-R est présent, à une concentration finale de 350µM, très inférieure au Km.

On incube BACE1 à 20 nM en présence de différentes concentrations de composés à tester de 1nM à 100µM (les concentrations d'inhibiteurs sont testées à partir de 100µM par dilutions au tiers), pendant 1 heure à 37°C, dans un tampon acétate de sodium/ acide acétique 100mM pH 4,5. La concentration finale de DMSO dans l'essai est de 10%.

Le pourcentage d'inhibition de l'activité de BACE1 par les composés de l'invention est déterminé en fonction de la quantité de produit de coupure détectée par HPLC. En effet, moins on détecte de produit de coupure, plus les composés ont une activité inhibitrice.

Les valeurs de Cl₅₀ sont ensuite calculées à partir des pourcentages d'inhibition précédemment déterminés, en fonction de la concentration en composés à tester.

Les composés de formule (I ter) selon la présente invention les plus actifs ont montré une Cl₅₀ inférieure à 50µM, de préférence inférieure à 10µM, de préférence inférieure à 1µM, de préférence inférieure à 100nM. Par exemple, le composé 69 du tableau a montré une Cl₅₀ de 2µM et le composé 53 du tableau une Cl₅₀ de 73nM.

### Test 2 Inhibition de la sécrétion cellulaire de β-A4 40 et APPbeta soluble avec une lignée cellulaire transfectée

Ce test utilise des cellules exprimant le substrat APP et BACE1.

Une lignée stable de cellules HEK293 [M. Citron et al, Nature, 1992, 360, 672-674] surexprimant le gène de l'APP751 portant la double mutation suédoise (Lys651 Met652 en Asn651 Leu652 ; numérotation de l'APP751) est utilisée. Les cellules sont distribuées à une densité de 15000 cellules par puits dans une plaque à 96 puits et cultivées pendant 72 heures dans un milieu de culture DMEM/Ham F12 contenant 10% de serum de veau foetal inactivé. Après renouvellement du milieu de culture, les composés à tester sont ajoutés à différentes concentrations (1 nM à 10µM) ; les concentrations d'inhibiteurs sont testées à partir de 10µM par dilutions au tiers]. La concentration finale de DMSO est de 1%. Les cellules sont cultivées pendant 24 heures en présence des composés à tester. La viabilité des cellules est contrôlée avec le kit MTS de Promega (G3581). Les surnageants sont collectés et les concentrations en peptides amyloide β-A4 40 et APPbeta swedish soluble sont déterminées en utilisant des kits de dosage commerciaux disponibles chez MSD (références K11 FTE-2 et K111 BUE-2). L'activité inhibitrice des composés à tester est calculée à partir du pourcentage d'inhibition de la sécrétion des peptides en fonction de la concentration en composé à tester.

### Test 3 Sélectivité vis-à-vis de la sécrétion cellulaire de APPalpha soluble avec une lignée cellulaire transfectée

Le même type d'essai que test 2 est utilisé.

### Test 4 Inhibition de la sécrétion cellulaire de β-A4 40 avec une lignée cellulaire naturelle

Une lignée commerciale (ATTC N°CCL-127) de neuroblastome humain, IMR32, est utilisée, exprimant naturellement le substrat APP et BACE1. Les cellules sont distribuées à une densité de 80000 cellules par puits dans une plaque à 96 puits et cultivées pendant 24 heures dans un milieu de culture DMEM/Ham F12 contenant 10% de serum de veau foetal inactivé. Après renouvellement du milieu de culture, les composés à tester sont ajoutés à différentes concentrations (1 nM à 10µM); les concentrations d'inhibiteurs sont testées à partir de 10µM par dilutions au tiers]. La concentration finale de DMSO est de 1%. Les cellules sont cultivées pendant 48 heures en présence des composés à tester. La viabilité des cellules est contrôlée avec le kit MTS de Promega (G3581). Les sumageants sont collectés et les concentrations en peptide amyloide β-A4 40 sont déterminées en utilisant le kit de dosage commercial disponible chez MSD (référence K11FTE-2). L'activité inhibitrice des composés à tester est calculée à partir du pourcentage d'inhibition de la sécrétion des peptides en fonction de la concentration en composé à tester.

Les résultats des tests biologiques montrent que les composés sont des inhibiteurs de la formation du peptide amyloïde (β-A4).

Ainsi, ces composés peuvent être employés dans le traitement des pathologies dans lesquelles un inhibiteur de la formation du peptide β-amyloïde (β-A4) apporte un bénéfice thérapeutique. Notamment de telles pathologies sont la démence sénile, la maladie d'Alzheimer, le syndrome de Down, la maladie de Parkinson, l'angiopathie amyloïde, les désordres cérébro-vasculaires, et/ou les maladies neurodégénératives (F. Coria et al, Neuropath. Appl. Neurobiol., 1996, 22,216-227).

L'utilisation des composés selon l'invention pour la préparation d'un médicament destiné à traiter les pathologies ci-dessus mentionnées fait partie intégrante de l'invention.

L'invention a également pour objet des médicaments qui comprennent un composé de formule (Iter), ou un sel d'addition de ce dernier à un acide pharmaceutiquement acceptable ou encore un hydrate ou un solvat du composé de formule (I ter). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou d'un sel pharmaceutiquement acceptable, d'un hydrate ou d'un solvat dudit composé, et éventuellement un ou plusieurs excipients pharmaceutiquement acceptables.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (Iter) ci-dessus, son sel, son solvat ou son hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Afin d'obtenir l'effet prophylactique ou thérapeutique désiré, la dose de principe actif peut varier entre 0,1 mg et 200 mg par kg de poids du corps et par jour. Bien que ces dosages soient des exemples de situation moyenne, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

Chaque dose unitaire peut contenir de 0,1 à 1000 mg, de préférence de 0,1 à 500 mg, de principe actif en combinaison avec un ou plusieurs excipients pharmaceutiques. Cette dose unitaire peut être administrée 1 à 5 fois par jour de façon à administrer un dosage journalier de 0,5 à 5000 mg, de préférence de 0,5 à 2500 mg.

La présente invention selon un autre de ses aspects, concerne également une méthode de traitement des pathologies ci-dessus indiquées qui comprend l'administration d'un composé selon l'invention, d'un sel pharmaceutiquement acceptable ou d'un hydrate dudit composé.

## Revendications

1. Composé de formule (Iter) : 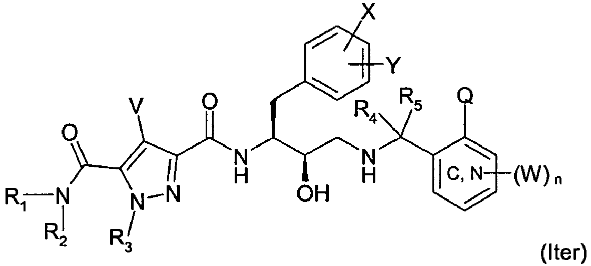 dans laquelle :
R₁ représente un atome d'hydrogène, un groupe C₃₋₆-cycloalkyle, C₁₋₆-alkyle, ledit C₁₋₆-alkyle étant éventuellement substitué par un ou plusieurs groupes choisis parmi un hydroxy, C₁₋₆-alcoxy;
• R₂ représente un atome d'hydrogène, un groupe C₁₋₁₀-alkyle, un phényle, lesdits groupe C₁₋₁₀-alkyle et phényle étant éventuellement substitués par un ou plusieurs groupes choisis parmi hydroxy, C₁₋₆-alkyle, C₁₋₆-alcoxy;
étant entendu que l'un au moins de R₁ ou de R₂ est différent d'un atome d'hydrogène ;
. ou R₂ représente un groupe NRₐR_{b} dans lequel :
Rₐ et R_{b} représentent, indépendamment l'un de l'autre, un atome d'hydrogène, un groupe C₁₋₆-alkyle, un groupe aryle, C₁₋₆-alkylène-aryle ou C₁₋₆-alkylène-hétéroaryle, lesdits groupe C₁₋₆-alkyle, aryle C₁₋₆-alkylène-aryle ou C₁₋₆-alkylène-hétéroaryle étant éventuellement substitués par un ou plusieurs groupes choisis parmi hydroxy, C₁₋₆-alkyle, C₁₋₆-alcoxy, halogène, CF₃ ou OCF₃,
Ou bien Rₐ et R_{b} forment ensemble avec l'atome d'azote auquel ils sont rattachés un hétérocycle, ledit hétérocyle étant éventuellement substitué par un ou plusieurs groupes choisis parmi C₁₋₆-alkyle, C₁₋₆-alkylène-O-C₁₋₆-alkyle ou C₁₋₆-alcoxy, halogène, CF₃ ou OCF₃;
. ou bien R₁ et R₂ forment ensemble avec l'atome d'azote auquel ils sont rattachés un groupe hétérocycle, ledit groupe hétérocycle étant éventuellement substitué par un ou plusieurs groupes choisis parmi C₁₋₆-alkyle, C₁₋₆-alcoxy, C₁₋₆-alkylène-O-C₁₋₆-alkyle ;
• R₃ représente un groupe C₁₋₆-alkyle ou un benzyle ;
• R₄ et R₅ représentent indépendamment l'un de l'autre un atome d'hydrogène, un groupe C₁₋₆-alkyle,
. ou bien R₄ et R₅ forment ensemble, avec l'atome de carbone auquel ils sont reliés un cycle spiro contenant de 3 à 5 atomes de carbone ;
• Q représente un atome d'hydrogène, un groupe C₁₋₃- alkyle ;
. ou bien Q et R₅ forment ensemble et avec la chaîne carbonée à laquelle ils sont rattachés, un cycle carboné saturé comportant 5 ou 6 atomes de carbone, ledit cycle étant éventuellement substitué par un ou deux groupes R, le ou les groupes R représentant indépendamment l'un de l'autre choisis parmi un atome d'halogène, un groupe C₁₋₃-alkyle, OCHF₂, OCF₃, CF₃ ou C₁₋₃-alcoxy ;
. ou bien Q et R₅ forment ensemble et avec la chaîne carbonée à laquelle ils sont rattachés, un cycle saturé comportant 4 ou 5 atomes de carbone et un atome d'oxygène, ledit cycle étant éventuellement substitué par un ou deux groupes R, le ou les groupes R représentant indépendamment l'un de l'autre choisis parmi un atome d'halogène, un groupe C₁₋₃-alkyle, OCHF₂, OCF₃, CF₃ ou C₁₋₃-alcoxy ;
• X et Y représentent indépendamment l'un de l'autre un atome d'hydrogène, d'halogène, un groupe -O-C₂₋₆-alkényle, ou -O-Benzyle ;
• n représente un nombre entier choisi parmi 1 ou 2 ;
• V représente un atome d'hydrogène ; et
• W représente un atome d'halogène, un groupe CF₃, C₁₋₆-alkyle, -COOH, C₁₋₆-alcoxy ;
étant entendu que lorsque n est 2, les deux groupes W représentent, indépendamment les uns des autres, les définitions mentionnées ci-dessus, dans laquelle :
- un groupe aryle est un système aromatique monocyclique ou polycyclique comprenant de 6 à 14 atomes de carbone, et
- un groupe hétérocycle est un groupe cyclique saturé de 4 à 7 chaînons comportant un à plusieurs hétéroatomes,
à l'état de base ou de sel d'addition à un acide, ainsi qu'à l'état d'hydrate ou de solvat, ainsi que ses énantiomères, diastéréoisomères et de leurs mélanges.

2. Composé de formule (Iquater) selon la revendication 1 : 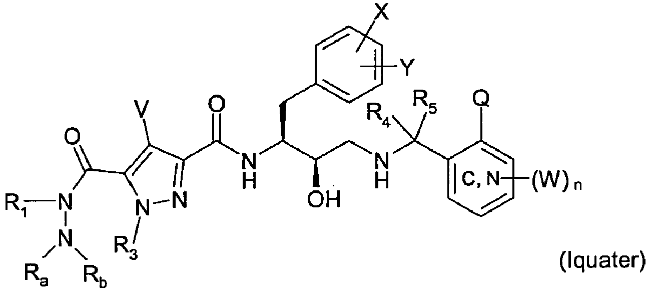 dans laquelle
Rₐ, R_{b}, R₁, R₂, R₃, R₄, R₅, X, Y, n, Q, V et W ont les mêmes définitions que les composés de formule (Iter),
à l'état de base ou de sel d'addition à un acide, ainsi qu'à l'état d'hydrate ou de solvat, ainsi que ses énantiomères, diastéréoisomères et de leurs mélanges.

3. Procédé de préparation d'un composé de formule (Iter) selon l'une quelconque des revendications 1 et 2, **caractérisé en ce que** l'on fait réagir un composé de formule (II) : 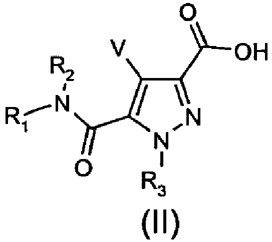 dans laquelle R₁, R₂, R₃ et V sont tels que définis pour le composé de formule (Iter) selon la revendication 1, dans un solvant inerte
avec un composé de formule (III): 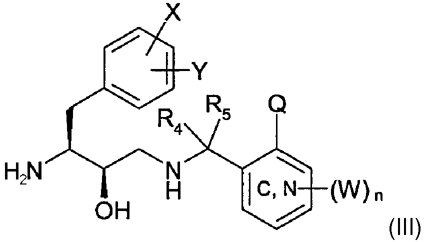 dans laquelle X, Y, R₄, R₅, Q , W et n sont tels que définis pour le composé de formule (Iter) selon la revendication 1, à une température pouvant varier de 0°C à la température ambiante,
pour donner le composé de formule (Iter) selon la revendication 1.

4. Médicament, **caractérisé en ce qu'**il comprend un composé de formule (Iter) selon l'une quelconque des revendications 1 et 2, ou un sel d'addition de ce composé à un acide pharmaceutiquement acceptable, ou encore un hydrate ou un solvat du composé de formule (Iter).

5. Composition pharmaceutique, **caractérisée en ce qu'**elle comprend au moins un composé de formule (Iter) selon l'une quelconque des revendications 1 et 2, ou un sel pharmaceutiquement acceptable, un hydrate ou un solvat de ce composé, ainsi qu'au moins un excipient pharmaceutiquement acceptable.

6. Composé de formule (Iter) selon l'une quelconque des revendications 1 et 2, pour son utilisation pour traiter la démence sénile, la maladie d'Alzheimer, le syndrome de Down, la maladie de Parkinson, l'angiopathie amyloïde, les désordres cérébro-vasculaires et/ou les maladies neurodégénératives.

## Patentansprüche

1. Verbindung der Formel (Iter): 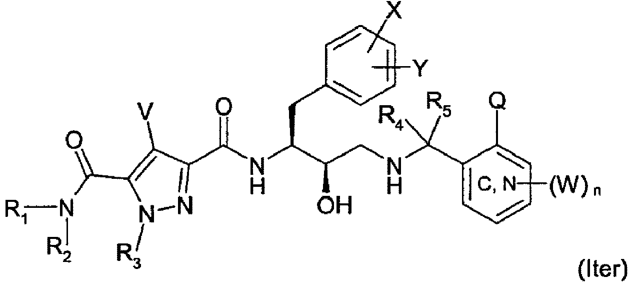 in der:
• R₁ für ein Wasserstoffatom, eine C₃₋₆-Cycloalkylgruppe oder eine C₁₋₆-Alkylgruppe steht, wobei das C₁₋₆-Alkyl gegebenenfalls durch eine oder mehrere aus Hydroxy und C₁₋₆-Alkoxy ausgewählte Gruppen substituiert ist;
• R₂ für ein Wasserstoffatom, eine C₁₋₁₀-Alkylgruppe oder eine Phenylgruppe steht, wobei die C₁₋₁₀-Alkyl- und Phenylgruppe gegebenenfalls durch eine oder mehrere aus Hydroxy, C₁₋₆-Alkyl und C₁₋₆-Alkoxy ausgewählte Gruppen substituiert sind;
mit der Maßgabe, dass mindestens einer der Reste R₁ und R₂ von einem Wasserstoffatom verschieden ist;
. oder R₂ für eine Gruppe NRₐR_{b} steht, in der: Rₐ und R_{b} unabhängig voneinander für ein Wasserstoffatom, eine C₁₋₆-Alkylgruppe oder eine Aryl-, C₁₋₆-Alkylen-aryl- oder C₁₋₆-Alkylen-heteroarylgruppe stehen, wobei die C₁₋₆-Alkyl-, Aryl-, C₁₋₆-Alkylenaryl- oder C₁₋₆-Alkylen-heteroarylgruppe gegebenenfalls durch eine oder mehrere aus Hydroxy, C₁₋₆-Alkyl, C₁₋₆-Alkoxy, Halogen, CF₃ und OCF₃ ausgewählte Gruppen substituiert ist;
oder auch Rₐ und R_{b} zusammen mit dem Stickstoffatom, an das sie gebunden sind, einen Heterocyclus bilden, wobei der Heterocyclus gegebenenfalls durch eine oder mehrere aus C₁₋₆-Alkyl, C₁₋₆-Alkylen-O-C₁₋₆-alkyl und C₁₋₆-Alkoxy, Halogen, CF₃ und OCF₃ ausgewählte Gruppen substituiert ist;
. oder auch R₁ und R₂ zusammen mit dem Stickstoffatom, an das sie gebunden sind, eine Heterocyclylgruppe bilden, wobei die Heterocyclylgruppe gegebenenfalls durch eine oder mehrere aus C₁₋₆-Alkyl, C₁₋₆-Alkoxy und C₁₋₆-Alkylen-O-C₁₋₆-alkyl ausgewählte Gruppen substituiert ist;
• R₃ für eine C₁₋₆-Alkylgruppe oder Benzyl steht;
• R₄ und R₅ unabhängig voneinander für ein Wasserstoffatom oder eine C₁₋₆-Alkylgruppe stehen,
. oder auch R₄ und R₅ zusammen mit dem Kohlenstoffatom, an das sie gebunden sind, einen Spirocyclus mit 3 bis 5 Kohlenstoffatomen bilden;
• Q für ein Wasserstoffatom oder eine C₁₋₃-Alkylgruppe steht;
. oder auch Q und R₅ zusammen und mit der Kohlenstoffkette, an die sie gebunden sind, einen gesättigten Kohlenstoffring mit 5 oder 6 Kohlenstoffatomen bilden, wobei der Ring gegebenenfalls durch eine oder zwei Gruppen R substituiert ist, wobei die Gruppe bzw. die Gruppen R unabhängig voneinander aus einem Halogenatom, einer C₁₋₃-Alkylgruppe, OCHF₂, OCF₃, CF₃ und C₁₋₃-Alkoxy ausgewählt sind;
. oder auch Q und R₅ zusammen mit der Kohlenstoffkette, an die sie gebunden sind, einen gesättigten Ring mit 4 oder 5 Kohlenstoffatomen und einem Sauerstoffatom bilden, wobei der Ring gegebenenfalls durch eine oder zwei Gruppen R substituiert ist, wobei die Gruppe bzw. die Gruppen R unabhängig voneinander aus einem Halogenatom, einer C₁₋₃-Alkylgruppe, OCHF₂, OCF₃, CF₃ und C₁₋₃-Alkoxy ausgewählt sind;
• X und Y unabhängig voneinander für ein Wasserstoff- oder Halogenatom oder eine -O-C₂₋₆-Alkenyl- oder -O-Benzylgruppe stehen;
• n für eine ganze Zahl steht, die aus 1 oder 2 ausgewählt ist;
• V für ein Wasserstoffatom steht und
• W für ein Halogenatom oder eine CF₃-, C₁₋₆-Alkyl-, -COOH- oder C₁₋₆-Alkoxygruppe steht;
mit der Maßgabe, dass dann, wenn n für 2 steht, die beiden Gruppen W unabhängig voneinander die oben angegebenen Definitionen repräsentieren,
wobei:
- eine Arylgruppe ein monocyclisches oder polycyclisches aromatisches System mit 6 bis 14 Kohlenstoffatomen ist und
- eine Heterocyclylgruppe eine gesättigte 4- bis 7-gliedrige cyclische Gruppe mit einem oder mehreren Heteroatomen ist,
in Basen- oder Säureadditionssalzform sowie in Hydrat- oder Solvatform sowie Enantiomere, Diastereoisomere und Gemische davon.

2. Verbindung der Formel (Iquater) gemäß Anspruch 1 : 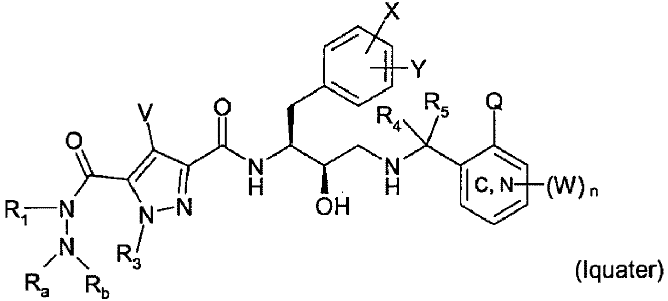 in der
Rₐ, R_{b}, R₁, R₂, R₃, R₄, R₅, X, Y, n, Q, V und W die gleichen Definitionen wie die Verbindungen der Formel (Iter) haben,
in Basen- oder Säureadditionssalzform sowie in Hydrat- oder Solvatform sowie Enantiomere, Diastereoisomere und Gemische davon.

3. Verfahren zur Herstellung einer Verbindung der Formel (Iter) nach einem der Ansprüche 1 und 2, **dadurch gekennzeichnet, dass** man eine Verbindung der Formel (II): 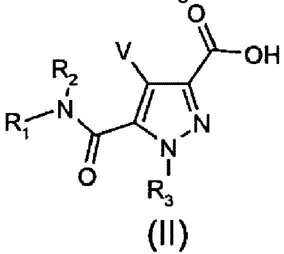 in der R₁, R₂, R₃ und V wie für die Verbindung der Formel (Iter) nach Anspruch 1 definiert sind, in einem inerten Lösungsmittel mit einer Verbindung der Formel (III): 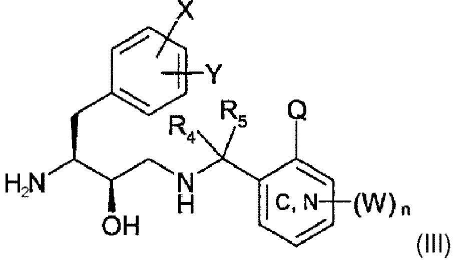 in der X, Y, R₄, R₅, Q, W und n wie für die Verbindung der Formel (Iter) nach Anspruch 1 definiert sind, bei einer Temperatur, die von 0°C bis Umgebungstemperatur variieren kann,
zu der Verbindung der Formel (Iter) nach Anspruch 1 umsetzt.

4. Medikament, **dadurch gekennzeichnet, dass** es eine Verbindung der Formel (Iter) nach einem der Ansprüche 1 und 2 oder ein Additionssalz dieser Verbindung mit einer pharmazeutisch unbedenklichen Säure oder auch ein Hydrat oder ein Solvat der Verbindung der Formel (Iter) umfasst.

5. Pharmazeutische Zusammensetzung, **dadurch gekennzeichnet, dass** sie mindestens eine Verbindung der Formel (Iter) nach einem der Ansprüche 1 und 2 oder ein pharmazeutisch unbedenkliches Salz, ein Hydrat oder ein Solvat dieser Verbindung sowie mindestens einen pharmazeutisch unbedenklichen Hilfsstoff umfasst.

6. Verbindung der Formel (Iter) nach einem der Ansprüche 1 und 2 zur Verwendung zur Behandlung von seniler Demenz, Alzheimer-Krankheit, Down-Syndrom, Parkinson-Krankheit, Amyloidangiopathie, zerebrovaskulären Störungen und/oder neurodegenerativen Erkrankungen.

## Claims

1. Compound of formula (Iter): 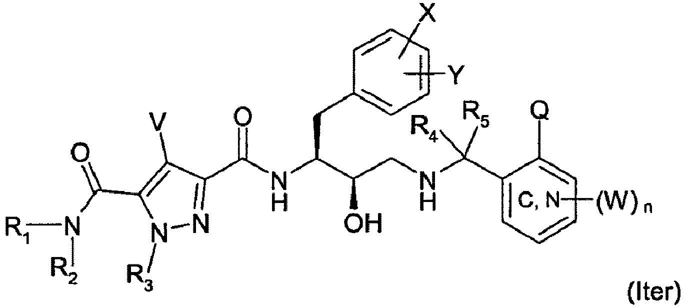 in which:
• R₁ represents a hydrogen atom, a C₃₋₆-cycloalkyl group, C₁₋₆-alkyl group, said C₁₋₆-alkyl optionally being substituted with one or more groups selected from a hydroxy, C₁₋₆-alkoxy;
• R₂ represents a hydrogen atom, a C₁₋₁₀-alkyl group, a phenyl group, said C₁₋₁₀-alkyl and phenyl groups optionally being substituted with one or more groups selected from hydroxy, C₁₋₆-alkyl, C₁₋₆-alkoxy;
it being understood that at least one of R₁ or of R₂ is different from a hydrogen atom;
. or R₂ represents a group NRₐR_{b} in which:
Rₐ and R_{b} represent, independently of one another, a hydrogen atom, a C₁₋₆-alkyl group, an aryl, C₁₋₆-alkylene-aryl or C₁₋₆-alkylene-heteroaryl group, said C₁₋₆-alkyl, aryl, C₁₋₆-alkylene-aryl or C₁₋₆-alkylene-heteroaryl group optionally being substituted with one or more groups selected from hydroxy, C₁₋₆-alkyl, C₁₋₆-alkoxy, halogen, CF₃ or OCF₃,
or alternatively Rₐ and R_{b} form, together with the nitrogen atom to which they are attached, a heterocycle, said heterocycle optionally being substituted with one or more groups selected from C₁₋₆-alkyl, C₁₋₆-alkylene-O-C₁₋₆-alkyl or C₁₋₆-alkoxy, halogen, CF₃ or OCF₃;
. or alternatively R₁ and R₂ form, together with the nitrogen atom to which they are attached, a heterocyclic group, said heterocyclic group optionally being substituted with one or more groups selected from C₁₋₆-alkyl, C₁₋₆-alkoxy, C₁₋₆-alkylene-O-C₁₋₆-alkyl;
• R₃ represents a C₁₋₆-alkyl group or a benzyl group;
• R₄ and R₅ represent, independently of one another, a hydrogen atom, a C₁₋₆-alkyl group,
• or alternatively R₄ and R₅ form, together with the carbon atom to which they are attached, a spirocycle containing from 3 to 5 carbon atoms;
• Q represents a hydrogen atom, a C₁₋₃-alkyl group;
• or alternatively Q and R₅ form together, and with the carbon chain to which they are attached, a saturated carbon ring having 5 or 6 carbon atoms, said ring optionally being substituted with one or two groups R, the group or groups R being, independently of one another, selected from a halogen atom, a C₁₋₃-alkyl, OCHF₂, OCF₃, CF₃ or C₁₋₃-alkoxy group;
• or alternatively Q and R₅ form together, and with the carbon chain to which they are attached, a saturated ring having 4 or 5 carbon atoms and an oxygen atom, said ring optionally being substituted with one or two groups R, the group or groups R being, independently of one another, selected from a halogen atom, a C₁₋₃-alkyl, OCHF₂, OCF₃, CF₃ or C₁₋₃-alkoxy group;
• X and Y represent, independently of one another, a hydrogen atom, a halogen atom, an -O-C₂₋₆-alkenyl, or -O-benzyl group;
• n represents an integer selected from 1 or 2;
• V represents a hydrogen atom; and
• W represents a halogen atom, a CF₃, C₁₋₆-alkyl, -COOH, C₁₋₆-alkoxy group;
it being understood that when n is 2, the two groups W represent, independently of one another, the definitions given above,
in which:
- an aryl group is a monocyclic or polycyclic aromatic system having from 6 to 14 carbon atoms, and
- a heterocyclic group is a saturated cyclic group with 4 to 7 ring members having one or more heteroatoms,
in the form of base or of salt of addition to an acid, as well as in the form of hydrate or of solvate, as well as its enantiomers, diastereoisomers and mixtures thereof.

2. Compound of formula (Iquater) according to Claim 1: 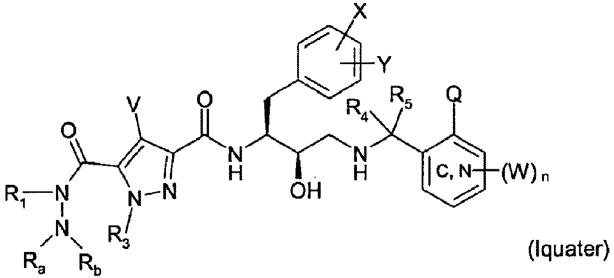 in which Rₐ, R_{b}, R₁, R₂, R₃, R₄, R₅, X, Y, n, Q, V and W have the same definitions as the compounds of formula (Iter),
in the form of base or of salt of addition to an acid, as well as in the form of hydrate or of solvate, as well as its enantiomers, diastereoisomers and mixtures thereof.

3. Method of preparation of a compound of formula (Iter) according to either of Claims 1 and 2, **characterized in that** a compound of formula (II): 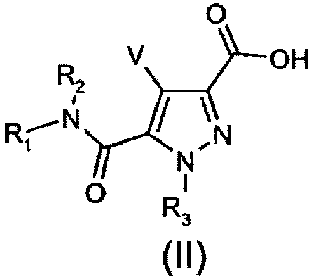 in which R₁, R₂, R₃ and V are as defined for the compound of formula (Iter) according to Claim 1, is reacted in an inert solvent with a compound of formula (III): 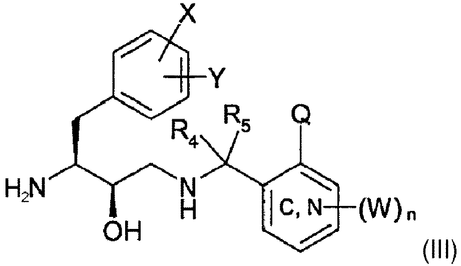 in which X, Y, R₄, R₅, Q, W and n are as defined for the compound of formula (Iter) according to Claim 1, at a temperature that can vary from 0°C to room temperature,
to give the compound of formula (Iter) according to Claim 1.

4. Medicinal product, **characterized in that** it comprises a compound of formula (Iter) according to either of Claims 1 and 2, or a salt of addition of this compound to a pharmaceutically acceptable acid, or a hydrate or a solvate of the compound of formula (Iter).

5. Pharmaceutical composition, **characterized in that** it comprises at least one compound of formula (Iter) according to either of Claims 1 and 2, or a pharmaceutically acceptable salt, a hydrate or a solvate of this compound, as well as at least one pharmaceutically acceptable excipient.

6. Compound of formula (Iter) according to either of Claims 1 and 2, for its use for the treatment of senile dementia, Alzheimer's disease, Down's syndrome, Parkinson's disease, amyloid angiopathy, cerebrovascular disorders and/or neurodegenerative diseases.
